(19) Europäisches Patentamt
European Patent Office
Office européen des brevets

(11) **EP 3 435 837 B1**

(12) **EUROPEAN PATENT SPECIFICATION**

(45) Date of publication and mention
of the grant of the patent:
**01.06.2022 Bulletin 2022/22**

(21) Application number: **17776717.5**

(22) Date of filing: **30.03.2017**

(51) International Patent Classification (IPC):
**A61B 1/303** (2006.01)   **A61B 1/012** (2006.01)
**A61B 1/05** (2006.01)   **A61B 1/06** (2006.01)
**A61B 1/307** (2006.01)

(52) Cooperative Patent Classification (CPC):
**A61B 1/303; A61B 1/00042; A61B 1/00052;
A61B 1/015; A61B 1/05; A61B 1/0607;
A61B 1/0676; A61B 1/0684; A61B 10/0291**

(86) International application number:
**PCT/US2017/025197**

(87) International publication number:
**WO 2017/173178 (05.10.2017 Gazette 2017/40)**

(54) **COLPOSCOPES**

KOLPOSKOPE

COLPOSCOPES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2016 US 201662315140 P**

(43) Date of publication of application:
**06.02.2019 Bulletin 2019/06**

(73) Proprietor: **Duke University
Durham, NC 27705 (US)**

(72) Inventors:
• **RAMANUJAM, Nirmala
Durham
NC 27705 (US)**
• **ASIEDU, Mercy
Durham
NC 27705 (US)**
• **LAM, Christopher
Durham
NC 27705 (US)**

• **MUELLER, Jenna
Durham
NC 27705 (US)**
• **AGUDOGO, Julia
Durham
NC 27705 (US)**
• **MIROS, Robert
San Rafael, CA 94901 (US)**

(74) Representative: **Yeadon IP Limited
Nexus
Discovery Way
Leeds LS2 3AA (GB)**

(56) References cited:
**WO-A1-2009/029254   WO-A1-2015/077684
US-A1- 2005 049 509   US-A1- 2008 058 605
US-A1- 2008 108 869   US-A1- 2009 062 662
US-A1- 2011 112 435   US-A1- 2013 066 165
US-B1- 6 277 067**

EP 3 435 837 B1

**Description**

TECHNICAL FIELD

The presently disclosed subject matter relates to colposcopes, inserters, and speculum-free imaging methods. Particularly, the presently disclosed subject matter relates to inserters having curved ends and associated methods.

BACKGROUND

Invasive Cervical Cancer (ICC) is the second most common female cancer in low and middle-income countries (LMICs) and the seventh most common in high-income countries. Annually, over 500,000 women are diagnosed, causing over 270,000 deaths recorded with more than 75% of cases occurring in Africa and India. The World Health Organization (WHO) estimates that currently 88% of worldwide ICC mortalities occur in LMICs, and this rate is expected to increase to 98% by 2030, furthering the disparities as the total number of annual worldwide mortalities increases to nearly 400,000. Though early diagnosis and treatment of cervical pre-cancers have been shown to significantly increase survival rates, diagnostic tools are not widely available in LMICs. Currently, as an alternative to cytology screening, the standard-of-care screening method in most LMICs is visual inspection with acetic acid (VIA), with or without digital image capture. This technique involves the use of a speculum to expand the vaginal canal to enable a clear field-of-view of the cervix, for visualization with a colposcope, camera or directly by the health provider (naked eye). Speculums are needed mainly because of the need to expand the entire vaginal canal.

During the VIA procedure, 3-5% acetic acid is applied to the surface of the cervix. A positive VIA exam shows a sharp, distinct, well-defined, dense aceto-white area, with or without raised margins. If a camera or digital colposcope is available, images of the cervix can be visualized at higher magnification and can also be archived for further analysis and review

Generally, colposcopes are stereomicroscopes with an extended working distance of between 250-350 mm. Working distance is defined as the length from the last optical element (outermost) to the target object. Colposcopes have 5 glass lens elements: common objective, zoom system (2 elements), erecting beam splitter, and eyepieces. A digital camera can be added with the use of a beam splitter between the right most zoom element and eyepiece. The mechanically compensated zoom lens systems allows for the working distance to remain constant when changing between magnifications, these 2 elements move independently. The colposcope lens elements are comprised of different glass materials of different refractive indices (crown and flint), with antireflective coating, and between 25 to 50 mm in diameter. Due to the complex arrangement and large number of elements of the traditional colposcope, the large device footprint, lack of portability due to weight, an expensive capital purchase, and reliance on walled electricity make wide scale dissemination and uptake difficult.

The speculum has been identified as a significant factor in the resistance of women to undergo cervical cancer screening, largely due to anxiety, fear, discomfort, pain, embarrassment, and/or vulnerability during the procedure. In the U.S., even though there is greater access to health care, compliance rates to cervical cancer screening vary, and embarrassment and fear of pain during examination have been reported as potential barriers to screening.

The speculum has been in existence in various shapes and forms since the tenth century and has evolved with hundreds of modifications in attempts to enhance exposure. The first rudimentary prototype of the modern speculum was developed out of a bent spoon. The semblance to the standard bivalve speculum was put forward by the manufacturer Charriere who introduced the bivalve, tri-blade and four-blade speculum. This inspired the duck bill designs of the familiar Cusco speculum in 1870 and the Graves speculum in 1878. These are cold, hard, metal devices with two bills each that expand the entire vaginal canal. Since the introduction of duck billed speculums, there have been few improvements to make them more comfortable and acceptable for women. Slight changes in design have involved introducing a variety of sizes and making the speculum out of plastic. Current speculums are designed for an external user, which makes it difficult for self-insertion by women. Being able to self-insert is important in being able to re-adjust when there is discomfort. Furthermore, in cases where women have tilted uteri or lax vaginal walls due to having a larger body size or a high parity, increased manipulation or use of an extra device, such as a side wall retractor, is needed to obtain a clear view of the cervix. This further adds to discomfort and pain during vaginal examinations.

The few attempts at major changes in the redesign of the speculum have been somewhat unsuccessful. The FemSpec, a clear plastic cylinder with inflatable air pockets, was developed in 2005 by FemSuite of San Francisco, California. The FemSpec has a tamponsized insertion diameter and, once inserted, can be inflated to expand the vaginal walls. This was taken off the market due to the reluctance of medical professionals to embrace the device. This device has been found to have sharp plastic edges and unable to withstand high vaginal pressures. The Vedascope, designed in Australia, is an encompassing speculum/colposcopic device, which dilates the vagina with air inflow and is attached to a camera and illumination for colposcopy. Though 92% of women have indicated a preference for the Vedascope to the speculum, it is very bulky, expensive and requires physician placement. Additionally, it has a potential risk for air embolism, which can be fatal.

WO 2009/029254 A1 describes an optical spectroscopic device for the identification of cervical cancer. WO 2015/077684 A1 describes a colposcope having light emitters and image capture devices. The colposcope has an elongate body and includes a balloon attached to the elongate body and configured to be inflated. US 2005/049509 A1 describes a cervix monitoring system with a probe and a monitoring unit. A head of the probe includes a camera and at least one light source.

In view of the foregoing, there is a need for improved speculum/colposcopic devices.

SUMMARY

There is provided a colposcope according to claim 1, with dependent claims 2 - 9 defining preferred embodiments thereof.

Disclosed herein are colposcopes, mammoscopes and speculum-free imaging methods (inserters) having curved ends and flat ends and associated methods. According to an aspect, an inserter includes an elongate body defining an interior space and having a distal end, a proximate end, and an axis extending between the distal end and the proximate end. The distal end is substantially funnel shaped and defines a wide portion and a narrow portion. The narrow portion is closer to the proximate end than the wide portion. An edge of a first portion of the wide portion extends further from the proximate end than an edge of a second portion of the wide portion.

According to another aspect, a speculum-free imaging device (inserter) is disclosed that has an elongate body defining an interior space and having a distal end, a proximate end, and an axis extending between the distal end and the proximate end. The distal end is substantially funnel shaped and defines a wide portion and a narrow portion. The narrow portion is closer to the proximate end than the wide portion.

According to other aspects, an imaging device for the visualization of body parts of a subject is disclosed. In certain aspects, the imaging device comprises a speculum-free imaging device (i.e., inserter) for imaging a body part of a subject, such as a cervix. The device is easy to use both by medical personnel on patients and by patients for self-examinations. In certain embodiments configured for vaginal imaging, it provides a clear wide field of view of the cervix comparable to that obtained from the speculum and is more comfortable and patient-oriented than the speculum. In such configurations, the device provided herein will be useful for cervical cancer screening but also for other applications, such as assessing ovulation, effacement of the cervix during labor and for educational purposes.

Another aspect of the present disclosure provides a method of using the speculum-free imaging device on a subject comprising, connecting the imaging device to the visualization component, inserting the device into the vagina of the device until the cervix comes into view, and viewing the images received by the image capture device on the visualization component.

In other examples not falling under the scope of claimed subject-matter, the elongate body and over-mold are removable from the image capture device and at least one light emitter.

Accordingly, one aspect of the present disclosure provides an imaging device (colposcope) comprising, consisting of, or consisting essentially of (a) an elongate body having a first end, a second defines an interior space; (b) an imaging capture device positioned within the interior space and positioned to capture images of an area outside the elongate body; (c) at least one light emitter attached to the first end of the elongate body and positioned to generate and direct light towards the are outside of the elongate body; and (d) an over-mold portion positioned at the first end and providing a water-resistant seal over the image capture device and at least one light emitter.

Another aspect of the present disclosure provides a method of using the imaging device as described herein on a subject comprising connecting the imaging device to the visualization component, placing the device next to the body part of interest, and viewing the images received by the image capture device on the visualization component.

According to the invention a colposcope with a control panel and associated electronics configured to select from among a plurality of modes of illumination for an object in a field of view of the image capture device is provided. The control panel and associated electronics are configured to select from among the modes of illumination to control the light emitter. According to the invention, the light emitter comprises a plurality of light emitting diodes (LEDs). The LEDs may be configured for white light imaging for VIA and VILLI, green light imaging for enhanced contrast for vasculature, or blue light imaging for excitation illumination of potential target fluorophores. The control panel and associated electronics may be configured to select from among a plurality of different magnifications or colors for viewing an object in a field of view of the image capture device.

In some examples not falling under the scope of claimed subject-matter, the over-mold further comprises a fog-resistant lens, the fog-resistant lens and cover mold provide a water-resistant seal.

In other embodiments, the imaging device further comprises a visualization component that is external to the elongate body and in communicative communication with the imaging capture device.

In other embodiments, the visualization component comprises a portable electronic device. In some embodiments, the portable electronic device comprises a laptop computer. In certain embodiments, the portable electronic device comprises a smartphone.

In other embodiments, the image capture device comprises a digital camera having a resolution of at least 2 MP. In certain embodiments, the image capture device comprises a digital camera having a resolution of at least 5 MP.

The working distance between the image capture device and fog-resistant lens is about 1 mm to about 50 mm. In another example not falling under the scope of claimed subject-matter, the working distance is about 10 mm to about 25 mm. In some examples not falling under the scope of claimed subject-matter, the working distance is about 20 mm to about 50 mm. In certain examples not falling under the scope of claimed subject-matter, the working distance is about 30 mm to about 50 mm.

In yet another example not falling under the scope of claimed subject-matter, the device comprises a viewing angle having a length of about 5 mm to about 40 mm. In other examples not falling under the scope of claimed subject-matter, the device comprises a viewing angle having a length of about 10 mm to about 35 mm. In another example not falling under the scope of claimed subject-matter, the device comprises a viewing angle having a length of about 15 mm to about 30 mm.

In yet another example not falling under the scope of claimed subject-matter, the device comprises a viewing angle having a length of about 20 mm to about 25 mm.

In some examples not falling under the scope of claimed subject-matter, the method further comprises aceto-whitening and/or staining for the presence of glycogen on the cervix for improved contrast imaging, the method further comprising prior to inserting the image device, inserting a cotton swab soaked in acetic acid or Lugol's iodine solution into the vagina such that the cervix is soaked in acetic acid and/or Lugol's iodine.

BRIEF DESCRIPTION OF THE SEVERAL VIEWS OF THE DRAWINGS

The foregoing aspects and other features of the present subject matter are explained in the following description, taken in connection with the accompanying drawings, wherein:

FIGs. 1A - 1E are different views of an example colposcope for imaging precancerous cervical lesions in accordance with embodiments of the present disclosure;

FIG. IF is a side cross-sectional view of an end cap for use with a colposcope in accordance with embodiments of the present disclosure;

FIGs. 1H - 1M are perspective views of example end caps in accordance with embodiments of the present disclosure;

FIGs 2A - 2E are various images showing experimental results conducted with example end caps in accordance with embodiments of the present disclosure;

FIG. 3 is a representative image of the fluorescent phantom imaged in high-resolution mode (3 micrometer resolution) with the scale bar at 100 micrometers;

FIG. 4 is an exploded view of another implementation of the device for imaging breast tumor margins topically stained with fluorescent contrast agents, which is referred to as the mammoscope in accordance with embodiments of the present disclosure;

FIG. 5 is a perspective view of a colposcope with speculum-free imaging device (Inserter) and an electronic device in accordance with embodiments of the present disclosure;

FIG. 6A is a perspective view of the inserter shown in FIG. 5;

FIG. 6B is a cross-sectional side view of the inserter shown in FIG. 6A;

FIGs. 6C and 6D are a perspective view and a cross-sectional side view, respectively, of another example inserter in accordance with embodiments of the present disclosure;

FIGs. 6E and 6F are a perspective view and a cross-sectional side view, respectively, of another example inserter in accordance with embodiments of the present disclosure;

FIGs. 7A - 7E are different views of another mechanical billed expander colposcope in a closed configuration and open configuration, respectively, in accordance with embodiments of the present disclosure;

FIGs. 8A - 8C are different views of a flat-tip inserter in accordance with embodiments of the present disclosure;

FIGs. 9A and 9B are cross-sectional side views showing using of a flat and curved end inserters, respectively, for manipulation of the cervix;

FIG. 10 shows images of a finite element analysis with factor of safety (FOS) plots over the entirety of different inserter/expander prototypes, ranging from relatively low values to high FOS values;

FIG. 11A shows representative images from each prototype as well as the standard Graves speculum of the cervix phantom at an untilted position under different pressures;

FIG. 11B shows a grouped bar plot of mean percent visual area of the cervix for different devices disclosed herein under different pressures;

FIG. 12A shows images from testing, after the tilted cervices had been manipulated towards the center position by the Graves speculum and both the flat tip and curved tip inserters;

FIG. 12B is a graph showing measured mean offset of the os from the center for each device under different uterus tilts;

FIG. 12C is a graph showing mean PVA for each device under different uterus tilts;

FIGs. 13A and 13B show representative images from a single volunteer with a sideverted cervix using the flat tip inserter and the curved tip inserter;

FIG. 13C is a graph showing visual results for a flap tip inserter and a curved tip inserter; and

FIG. 13D is a graph showing measured offset of OS from center after manipulating cervix for a flap tip inserter and a curved tip inserter.

DETAILED DESCRIPTION

For the purposes of promoting an understanding of the principles of the present disclosure, reference will now be made to various embodiments and specific language will be used to describe the same. It will nevertheless be understood that no limitation of the scope of the disclosure is thereby intended, such alteration and further modifications of the disclosure as illustrated herein, being contemplated as would normally occur to one skilled in the art to which the disclosure relates.

Unless otherwise defined, all technical terms used herein have the same meaning as commonly understood by one of ordinary skill in the art to which this disclosure belongs.

Articles "a" and "an" are used herein to refer to one or to more than one (i.e. at least one) of the grammatical object of the article. By way of example, "an element" means at least one element and can include more than one element.

In accordance with embodiments, the present subject matter relates to colposcopy. For example, inserters are described herein that utilize the principle of a mechanical delivery method for insertion and stabilization into the vagina for imaging of the external cervix. The imaging may produce digital, color, high-resolution images at both full field and at high magnification of areas of interest. Inserters described herein may include an image capture device for capture and storage of high-resolution, multimodal images of the external cervix for post-hoc analysis by medical personnel at a centralized location.

In accordance with examples not falling under the scope of claimed subject-matter, an inserter, a colposcope and electronic device may be a part of a kit provided for use by medical personnel to allow for screening of patients. Captured images may be suitably stored and processed. In an example, the images may be communicated or downloaded to a server for remote expert diagnosis. The colposcope may be suitably sterilized and subsequently re-used.

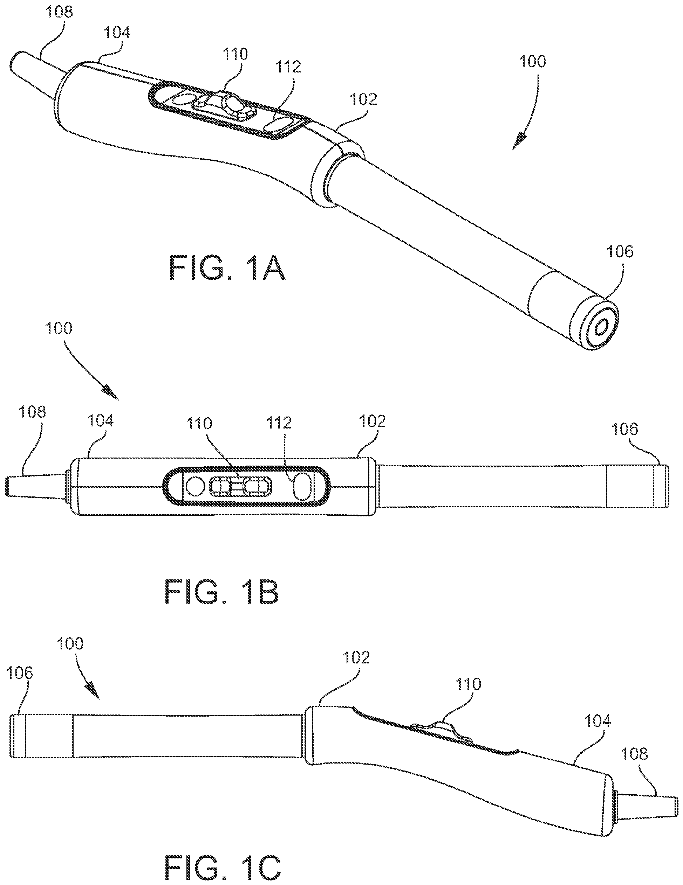

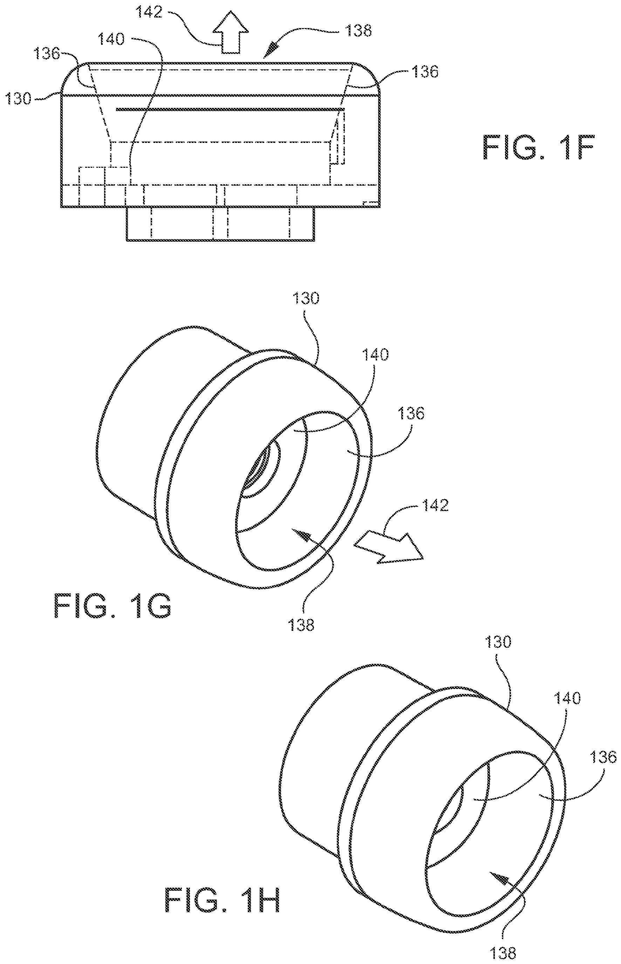

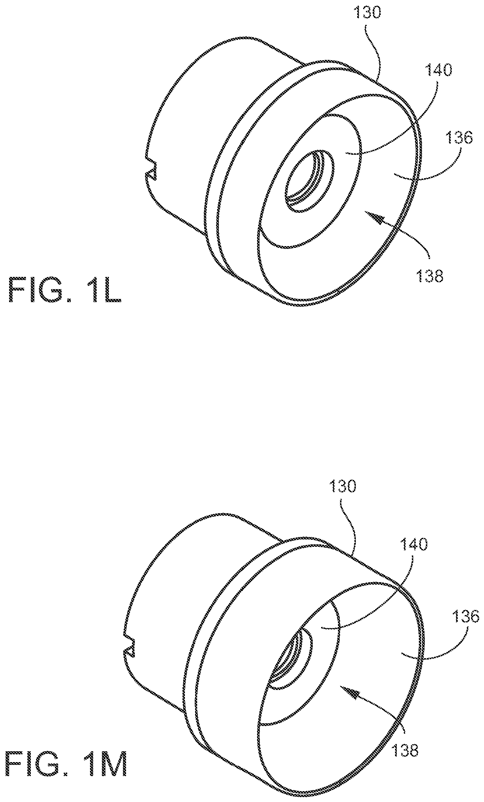

FIGs. 1A- 1E illustrates different views of an example colposcope 100 for imaging precancerous cervical lesions in accordance with embodiments of the present disclosure. Referring to FIG. 1A, the figure shows a perspective view of the colposcope 100 including a handle 102 at a proximate end 104. The colposcope includes a camera (e.g., a 5 MP camera) (132) and white, green, and blue light emitting diodes (LEDs) (not shown) functionally integrated into a distal end 106. A glass optical long pass filter 505 nm (housed in 131) is used to remove the excitation light (blue LEDs) for the fluorescent compound and only allow higher fluorescent light to return to the camera, but not interfere with white and green illumination modes. In an example, the colposcope 100 weigh less than 1 pound. The colposcope 100 also includes an electronic cord 108 for connection and interface to an electronic device, such as various electronic devices for control and presentation as described herein. The electronic cord 108 may also provide power to the device for enabling imaging and other functions. FIG. 1B illustrates a top view of the colposcope 100. FIG. 1C illustrates a side view of the colposcope.

The handle 102 includes a control panel having a slider 110 and a button 112 for control of operation of the colposcope 100. Particularly, the slider 110 can be moved by a user to control the working distance, field of view (FOV) and resolution. The button 112 can be pushed to enable switching between illumination with either a white or green LED. A button on the device enables switching between illumination with a white or green LED. The diagonal FOV and resolution at a 35 mm working distance is 34 mm and 22 $\mu$m, and at a 10 mm working distance is 5 mm and 3 micrometers.

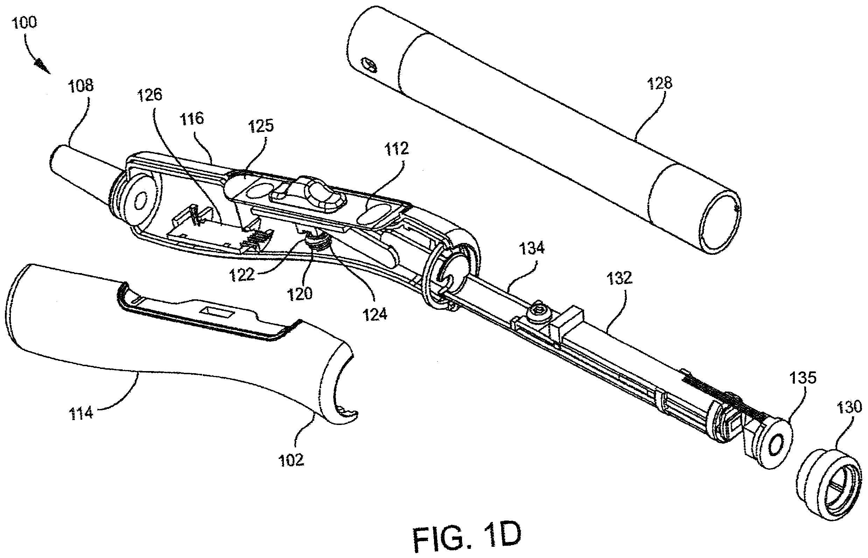

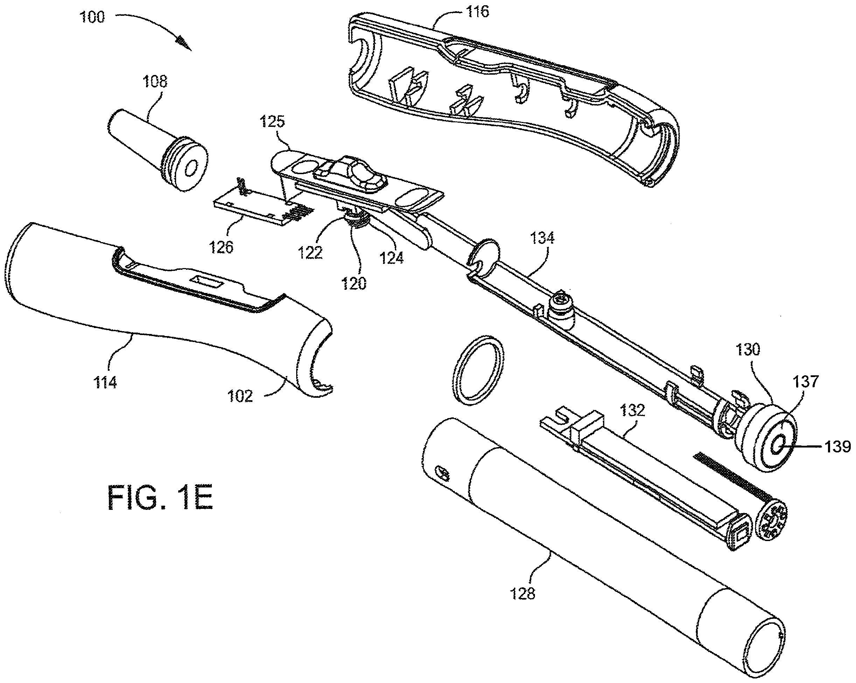

FIGs. 1D and 1E illustrate different exploded views of the colposcope 100. Referring to FIGs. 1D and IE, the handle 102 of the colposcope 100 is formed at least partially by a housing that is put together by housing parts 114 and 116. The housing parts 114 and 116 can be connected together to form the handle 102 and house the electronic and mechanical components for operation of the slider 110 and the button 112. Particularly, the slider's 110 movement is operable by use of the retainer assembly, which is comprised of pin 120, washer 122 and spring 124. The compression forces produced by this assembly provide a liquid and dust tight seal without inhibiting the freedom of movement. Specifically, the retainer assembly enables the easy sliding motion of slider 110 and enables connection to sled 134, which allows the user to zoom in on different features of the cervix, while also providing a tight seal preventing liquid and particle infiltration into the handle. The housing may also be formed by an interface panel 125 that is operatively connected to the slider 110 and the button 112. The housing clamshell housing is ultrasonically welded to ensure liquid and dust tight protection and doesn't depend on medical superglue or epoxy.

The housing may also contain a printed circuit board (PCB) and LED driver board 126 for implementing functionality as described herein Specifically, the LED driver board 126 allows the manufacturer to program the lux levels of the three LED settings, low white, high white, green, blue light. The LED driver board can allow the user to switch between the three LED settings, low white, high white, blue, and green light to image acetic acid staining, Lugol's iodine staining, and vascular patterns in the organ tissue bed of interest (e.g. cervix or breast). With continuing reference to FIGs. 1D and IE, the distal end of the colposcope 100 may be formed of a sleeve 128 and an end cap 130. The sleeve 128 and the end cap 130 may be fitted together. APCB and camera 132 may be held inside the sleeve 128. A holder 135 may also be fitted inside the sleeve 128 for carrying various components, including the PCB and camera 132. The distal end includes a light guide and diffuser 137 located within the end cap 130. The geometry of the light guide is significant in that the 3D design prevent image vignetting and eliminate environmental or stray light eliminating baffle and structurally supports the concentric LED PCB ring. A hydrophobic window 139 is mated to center of the light guide and diffuser 137 by a press fit and reinforced by a silicone O-ring and medical grade epoxy to prevent liquid and particle infiltration. Imaging quality is not compromised with the addition of these structural components validated by computational ray tracing and empirical validation testing.

The end cap 130 may be suitably configured for illuminating an object to be imaged. For example, FIG. IF illustrates a side cross-sectional view of an end cap 130 for use with a colposcope, such as colposcope 100 show in FIGs. 1A - 1E, in accordance with embodiments of the present disclosure. FIG. 1G illustrates a perspective view of the end cap 130 shown in FIG. IF. Referring to FIGs. IF and 1G, the end cap 130 may include a reflective surface 136 that extends around edges of a recess 138 within the end cap 130. The reflective surface 136 may be made of any suitable reflective material, such as a metal. The reflective surface 136 may be positioned and configured to receive light emitted by light emitters situated within the end cap 130. For example, the light emitters may be one or more LEDs positioned along a surface 140 for emitting light in a direction generally indicated by arrow 142. It is noted that the direction indicated by arrow 142 is also the general direction of a field of view of the image capture device (e.g., camera) of the colposcope. Thus, the light emitters are positioned to illuminate objects within the field of view of the light capture device.

In the example of FIGs. IF and 1G, an end cap is shown having a reflective surface 136 angled at 75 degrees with respect to an axis of the body of the colposcope. Further, the length of the reflective surface is 4.5 millimeters. The reflective surface 136 can refocus light normally lost outside the region of interest in a uniform matter.

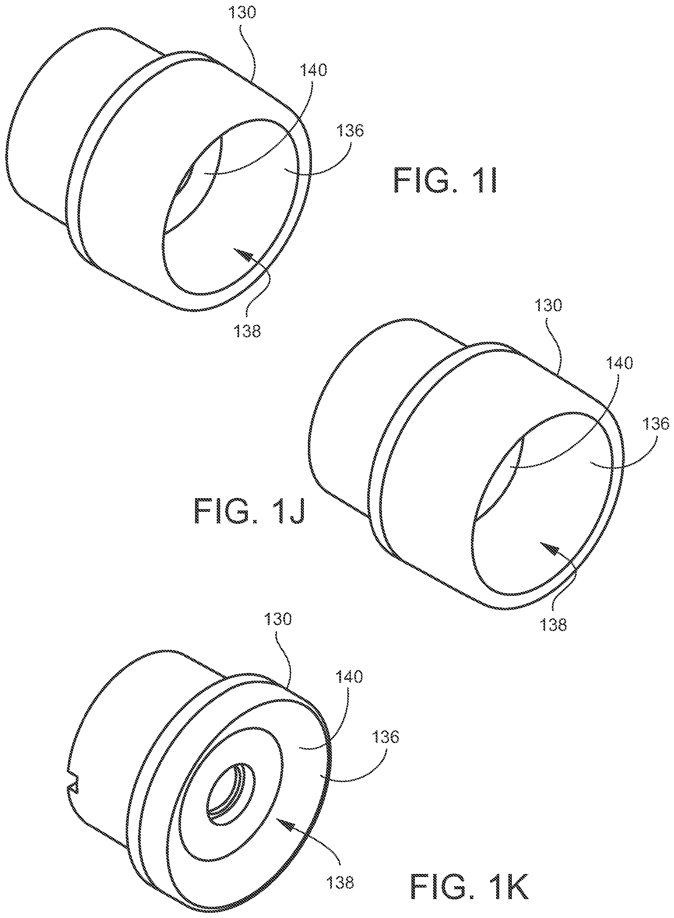

In alternative embodiments of the end cap, the reflective surface of an end cap may be configured differently than the reflective surface 136 shown in FIGs. IF and 1G. For example, FIGs. 1H - 1M illustrate perspective views of other example end caps 130 in accordance with embodiments of the present disclosure. Particularly, FIG. 1H shows an end cap 130 have a reflective surface 136 angled at 75 degrees and a length of 5.5 millimeters. FIG. 1I shows an end cap 130 have a reflective surface 136 angled at 75 degrees and a length of 6.5 millimeters. FIG. 1J shows an end cap 130 have a reflective surface 136 angled at 75 degrees and a length of 7.5 millimeters. FIG. 1K shows an end cap 130 have a reflective surface 136 angled at 30 degrees and a length of 1.8 millimeters. FIG. 1L shows an end cap 130 have a reflective surface 136 angled at 60 degrees and a length of 5.5 millimeters.

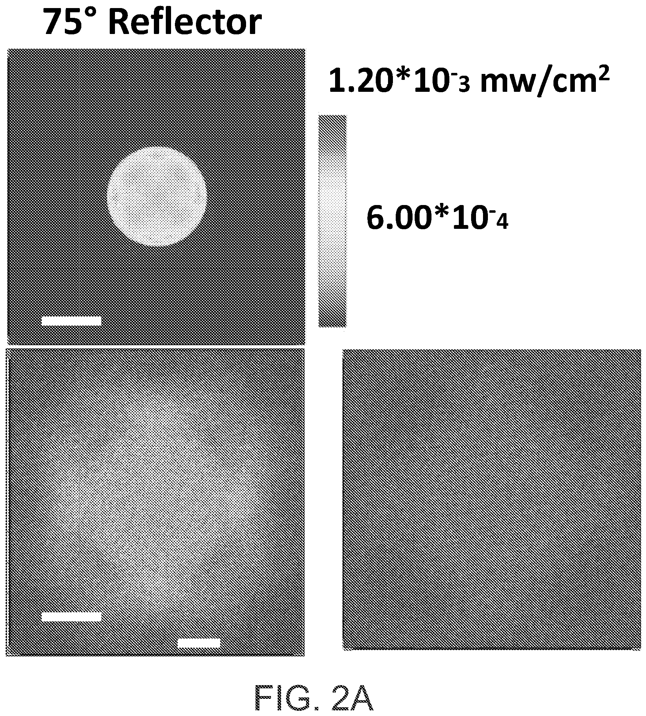

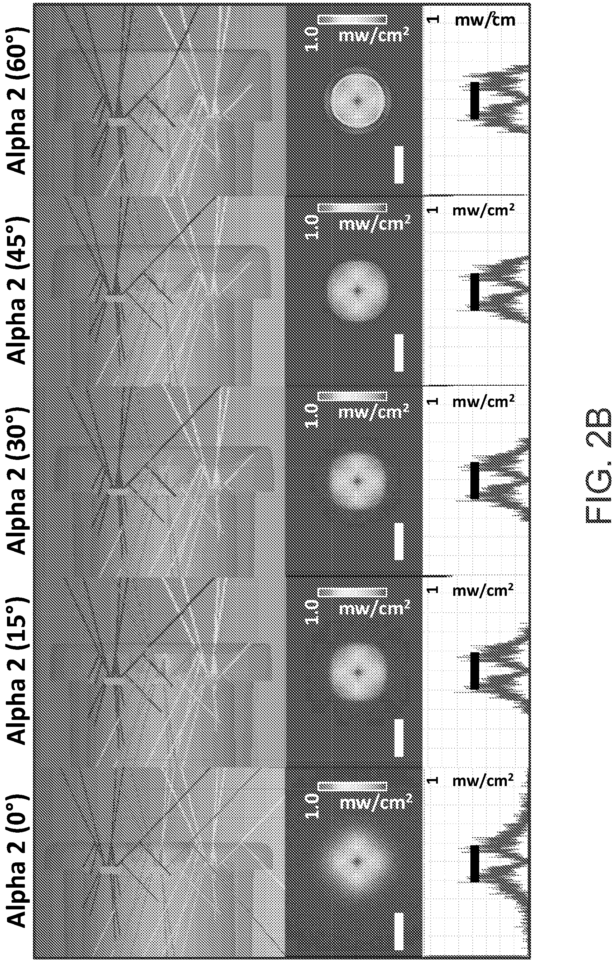

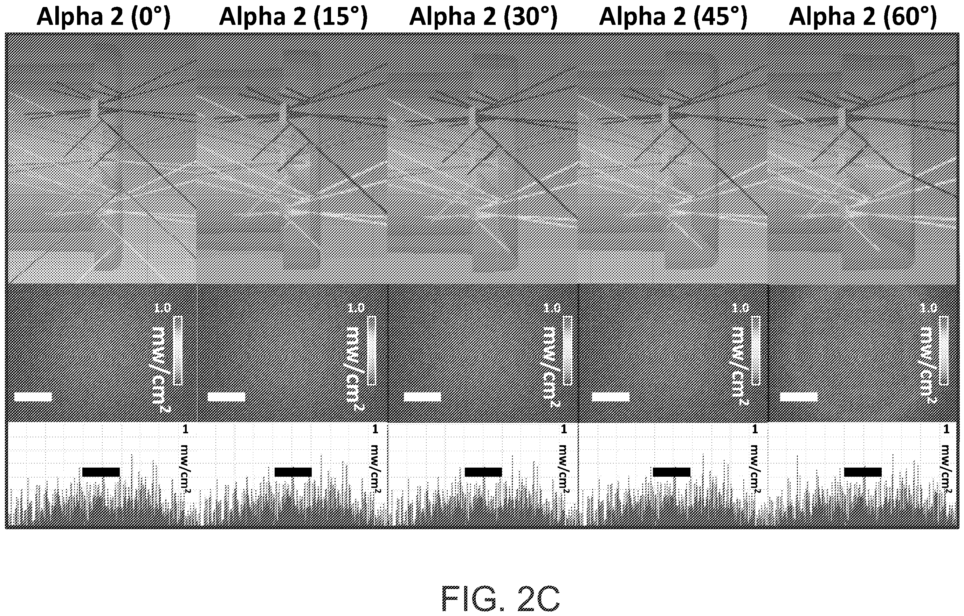

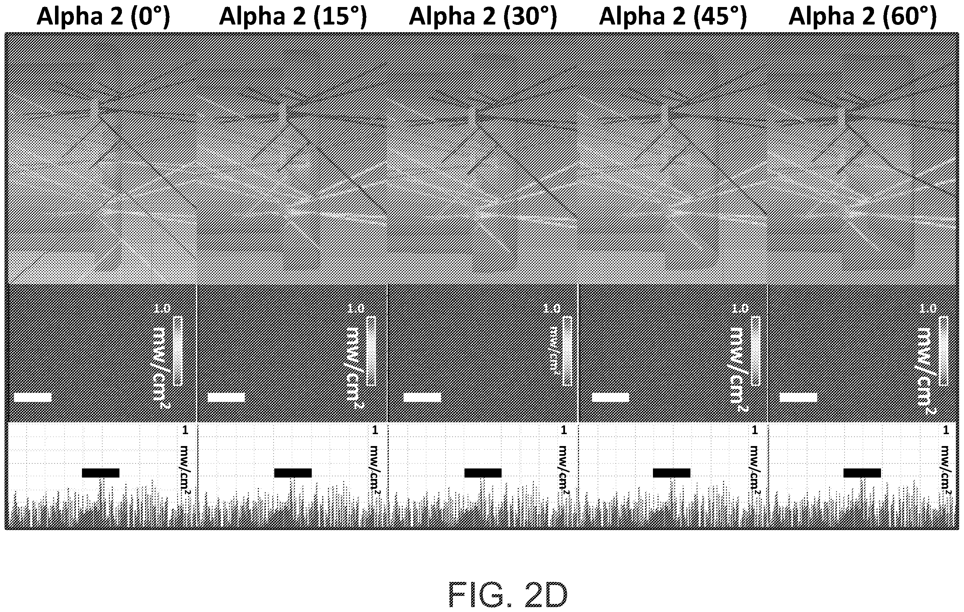

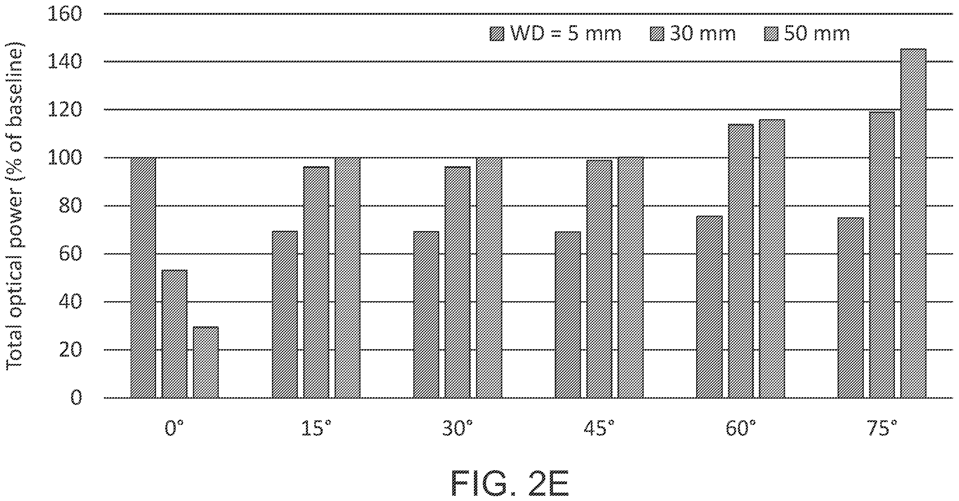

Experiments were conducted with end caps as shown in FIGs. 1G - 1M. FIGs 2A - 2E are various images showing results of such experiments, where the reflector improves the beam uniformity across working distances when compared to the bare and lower angle reflectors.

In accordance with embodiments, the presently disclosed colposcopes and techniques may be used for other indications, such as imaging breast tumor margins and oral cancer. The colposcope may be altered to function as a mammoscope by incorporating fluorescence imaging capabilities of an exogenous contrast agent (e.g. anthracyclines (a family of antibiotics that binds to dsDNA) and doxycycline (a family of antibiotics that bind to mitochondrial ribosomes) and fluorescently tagged receptors or inhibitors, as potential vital stains. Colposcopes and techniques disclosed herein may be used for the effective visualization and treatment of residual disease at the time of an initial breast conserving surgery (BCS) while minimizing risks of re-excision surgeries and radiation and the cost of repeat visits and interventions. In accordance with embodiments, the primary tumor or the tumor cavity can be rapidly assayed for the presence of residual disease. The tumor cells can be selectively visualized using a fluorescently labeled agent that when topically applied targets a ubiquitous signaling node common to the all subtypes of HPV induced cancer and to exploit metabolic differences in all types of breast cancer, including ductile carcinoma in situ (DCIS). The tumor cells may be localized by easily navigating back and forth between wide-field (to maximize sensitivity) and high-resolution imaging (to maximize specificity). The agent can be designed to have a dual role of selectively targeting tumor cells, at a low dose and demonstrating therapeutic potency at a high dose. This can allow for the same agent to eradicate residual tumor cells when applied topically to the tumor bed for those patients with residual disease. The etiology of cervical cancer by Human Papilloma Virus (HPV) a double stranded (ds)DNA virus, was first postulated by Dr. Harald zur Hausen in 1977 and later confirmed by 1984 with the extraction of integrated and episomal HPV dsDNA from cervical neoplasia, carcinoma, and immortalized cervical cancer cell lines. HPV are a family of viruses with circular dsDNA around 8kbp in length, an icosahedral nucleo-capsid shell with a diameter between 45-55 nm. In cervical cancer, the target cell for infection by HPV are the basal keratinocytes bottommost layer of the cervical squamous epithelium and uniquely the only normally undergo cell replication. After cellular division at the basal layer, the keratinocytes begin to rise towards the surface of the cervix and undergo differentiation process that involves flattening of the cell but no additional mitosis flattening concurrent with their differentiation where when infected the virion will be duplicated up to 1000 times before being released to infect other cells. These infected cells lose their ability to terminally differentiate. The thickness of epithelial layer is reported to be 260 to 450 $\mu$m and does not significantly change with disease severity. HPV oncogene products have been shown to mediate the normal regulatory apoptosis cascade, leading to uncontrolled proliferation and lack of terminal differentiation. In the mid 1960's investigations by EM (electron microscopy) revealed some curious findings: abnormal number of chromosomes in the nucleus (now believed to be episomal HPV copies), a significant increase in non-membrane associated ribosomes that aggregate together, and a significant increase in RER (rough endoplasmic reticulum) in early cervical neoplasia when compared to normal control tissue. These structural findings suggest viral hijacking of internal organelles of infected cells for production of oncoproteins, HPV virion duplication, and production of the capsid casing for the HPV virions prior to dispersion. Patients that were HPV-16+ and with biopsy confirmed high grade precancerous lesions (HSIL/CIN2+) had on average a 60 and 30 fold higher number of viruses per cell when compared to HPV+ women with biopsy confirmed normal cervices and biopsy confirmed low grade (LSIL/CIN1) cervices, respectively. Thus, a potential potent early surveillance target for cervical neoplasia could be selective staining of dsDNA for the episomal presence of HPV and increased dsDNA due to increased rates of uncontrolled cellular division induced by HPV infection at the superficial layer of the cervix where mitosis doesn't normally occur.

Concordant with the replication of virus and controlled cellular replication, there is a dramatic increase in energy consumption by the HPV infected cervical cells. Upregulation of glucose (glucose transporter, GLUT-1) and lactate (monocarboxylate transporters, MCT-1 and MCT-4) transporters has been widely reported in cervical pre-cancers and cancers with increasing expression highly correlated with increasing severity of disease. Increased activity of the first two enzymes in the pentose pathway, 6-phosphogluconate dehydrogenase and glucose-6-phosphate dehydrogenase has been reported in cultured samples from patients with biopsy confirmed cervical intraepithelial neoplasia and confirmed with studies in HPV+ cervical carcinoma cell lines, indicating the Warburg effect on the metabolism towards aerobic glycolysis. HPV-16E7 oncoproteins increased glycolysis and glutaminolysis through upregulation of pyruvate kinase type M2. Studies in human keratinocytes cell lines infected by HPV demonstrated a profound increase in energy consumption when compared to non-infected control cells. This increased demand in energy was concordant with an increased protein translation and virion duplication, followed by contraction of cell size and shift to IRES (internal ribosome entry site) dependent translation for production of proteins. Furthermore, primary cervical cells immortalized by HPV infection demonstrated markedly higher rates of estrogen (16$\alpha$-hydroxylate estradiol) and cholesterol metabolism when compared to normal control cell lines, potentially indicative of additional virally induced metabolic adaptations. More recently, the mechanism for this metabolic adaptation has been further elucidate with the discovery that the E2 oncoproteins encoded by high risk HPV-16 and -18, but not low risk HPV-6 was shown to co-localize with the host cell's mitochondria and induce distinct cristae morphology changes concordant with a significant increase in mitochondrial reactive oxygen species (ROS) production without the induction of apoptosis. Furthermore, the E1^E4 oncoproteins encoded by high risk HPV-16 has been shown to first bind to and initiate the breakdown of the cytokeratin support network of the infected cell and then is translocated to the mitochondria. The mitochondria bound to E1^E4 begin to migrate and aggregate around the nucleus before depolarizing and initiation apoptosis. This is the hypothesized mechanism for the release of the duplicated HPV virion once the cervical epithelial cell reaches to outermost epithelial surface to infect other cells. Furthermore, there is a profound and significant association for the increasing number of mitochondrial DNA mutations with severity of cervical pre-cancer and cancer grade. Increases in mitochondrial DNA copy number have been associated with higher risk of other cancers including: breast, head and neck, endometrial, ovarian, among others. Although, it's unknown if these mutations occurred prior to HPV infection or as a result of HPV infection's increased mitochondrial ROS production. Each mitochondrion is estimated to contain between 2 to 10 copies of mitochondrial (mt)DNA, which is also a circular dsDNA like HPV There are approximately 1000 total copies of mtDNA per normal human cell. Thus, selective staining of mitochondria to assess metabolic function and/or dsDNA content would enable earlier surveillance of cervical neoplasia. Increased inner mitochondrial membrane potential ($\Delta\Psi$m) as measured by higher rates of sequestration and accumulation of fluorescent cationic lyophilic dyes (e.g. Rhodamine 123, TMRE/TMRM, JC-1) has been widely reported in carcinoma and other metabolically active normal cells undergoing mitosis. Mitochondrial DNA (mtDNA) copy number and total mitochondrial mass as assessed by inner mitochondrial membrane independent stains (i.e. 10-N nonyl-acridine orange which binds to cardiolipin an inner mitochondrial membrane protein) has been shown to increase significantly in response to oxidative stress. Another fluorescent stain for mitochondrial mass, from the antibiotic tetracycline family, has been shown to preferential stain malignant tissue more heavily than normal tissue in squamous cell, non-melanoma skin, gastric, laryngeal, pharyngeal, and oral cancers among others. The cytoplasmic fluorescence distribution of tetracycline is consistent with the mitochondrial ribosome 30s subunit by allosteric inhibition of the amino acyl-tRNA at the acceptor site preventing protein synthesis. A phenomena first reported in 1960's where tetracycline inhibited the translation of proteins encoded by mitochondria (mtDNA), but not by nuclear DNA (nDNA) in yeast. Interestingly, this homology and potential bacterial origin of mitochondria leads to similar disruption of the mitochondrial protein synthesis process in humans that could provide a unique avenue for targeting cancer cell via their unique metabolic profile. Thus, selective staining of mitochondria through visualization of the increases in inner mito-

chondrial membrane potential($\Delta\Psi$m), increases in mtDNA (dsDNA) concentration, or increases mitochondrial mass (assessed by cardiolipin or mitochondrial ribosome staining) might prove an important early surveillance tool for improving cervical neoplasia detection accuracy.

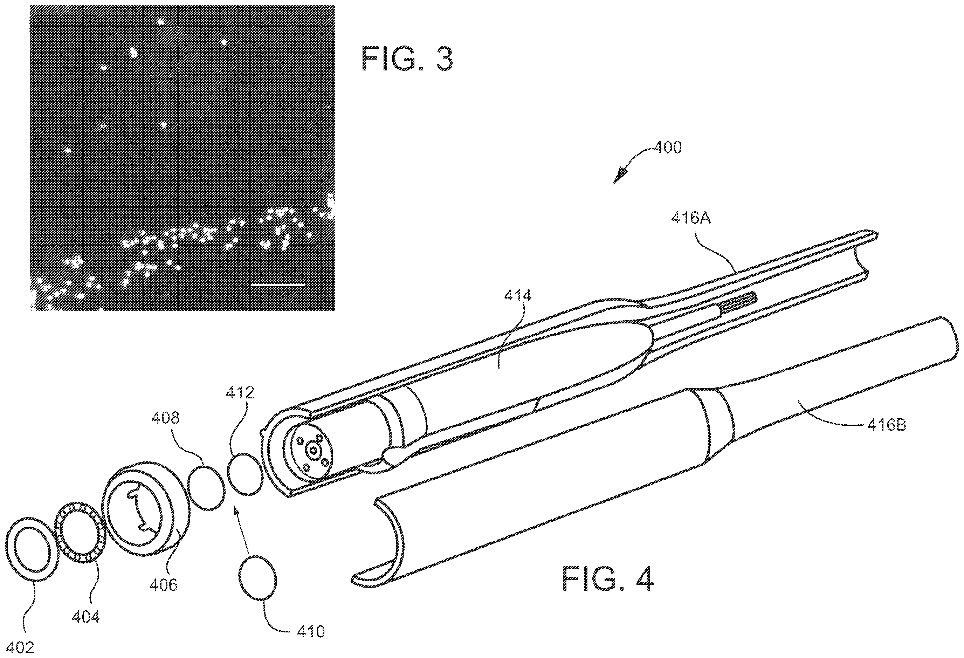

In accordance with embodiments, a colposcope as disclosed herein can be modified for use as a mammoscope. In an example, the green LEDs at the distal end of the probe can be replaced with blue LEDs (470/490 nm) to excite a variety of fluorophores. A band pass filter can be added in front of the camera to ensure only fluorescence reaches the camera (see e.g., FIG. 4), without cutting off any of the illumination from the blue LED. FIG. 4 is an exploded view of another example mammoscope 400 in accordance with embodiments of the present disclosure. Referring to FIG. 4, the mammoscope 400 includes a linear film polarizer 402, a white and blue concentric LED ring 404, an aluminum LED ring heat sink and optic mount 406, an anti-reflection coated hydrophobic window 408, a green bandpass filter 410, a linear glass polarizer 412, a 5-megapixel CMOS camera 414 with autofocus and USB 2.0, (416A, 416B) probe handle.

In other experiments, 10 $\mu$m diameter fluorescent beads on a glass slide (simulating a superficial layer of fluorescence in tissue) with a mammoscope in accordance with embodiments disclosed herein. Representative images collected at a 10 mm working distance demonstrate the fluorescence imaging capabilities. For example, FIG. 3 is a representative image of the fluorescent phantom imaged in high-resolution mode (3 micrometer resolution) with the scale bar at 100 micrometers.

In accordance with embodiments, the disclosed subject matter may be used to establish wide-field, high-resolution, visualization strategies, automated segmentation algorithms, and a fluorescently antibiotics to enhance specificity of the system. In an example, a colposcope having features disclosed herein may be adapted to function as a mammoscope. The mammoscope may be a portable wide-field, high-resolution imaging system utilizing a fluorophore for visualization and treatment of residual disease during BCS. The sensitivity and specificity of the mammoscope to detect fluorescence may be established as a function of progressively decreasing tumor cell density in a pre-clinical model of breast cancer.

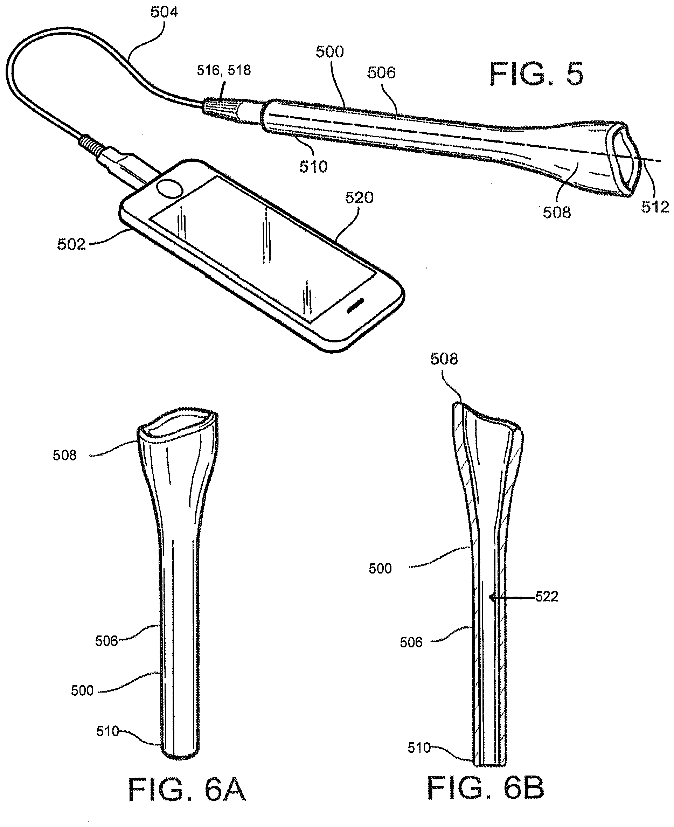

FIG. 5 illustrates a perspective view of a speculum-free imaging device (Inserter with colposcope) 500 and an electronic device 502 in accordance with embodiments of the present disclosure. Referring to FIG. 5, the colposcope within the inserter 500 and electronic device 502 are communicatively connected by a cable 504. In this example, the colposcope 500 and electronic device 502 communicate in accordance with the universal serial bus (USB) standard. Alternatively, the inserter 500 and electronic device 502 may communicate by another suitable communications standard.

The inserter 500 includes an elongate body 506 having a distal end 508, a proximate end 510, and an axis indicated by broken line 512. The body 506 is generally tubular and rounded in shape. Alternatively, the body 506 may be of any suitable shape and size.

Referring to the distal end 508, this end has a curved funnel-like tip. The curved tip enables easy manipulation of the cervix, especially in cases where the patient has a tilted uterus. In this example, the device has a slim tubular body that can range between 1 centimeter to 1.5 centimeters to enable accommodation of the camera through the channel while still preserving patient comfort. The tubular body opens up to a curved funnel-like tip which can range between about 2.5 centimeters and about 3.5 centimeters in diameter (range of anatomical cervix diameter). The tip may be curved (a variable s-curve) and funnel like for enabling a fit with the curvature of the cervix and protrudes on one end to scoop the cervix in place can function the same way.

For testing, each device was rapid prototyped using a 3D printer (dimension 1200es, Stratasys, Ltd.). The mechanical expansion device was assembled using stainless steel pins. The silicone device was assembled by attaching a menstrual cup to a 3D printed cylindrical stem. For bench testing, a custom-made vaginal phantom was created using a synthetic female reproductive organ. The organ included an outer genitalia, labia, a vagina, cervix, inner and outer os (cervical opening into the uterus), uterus, ovaries and fallopian tubes. The structural design was based on an amalgam of CT and MRI images from actual patients and the synthetic tissues employed in construction had been validated against the mechanical, physicochemical, thermal and dielectric properties of living tissue. The organ was compatible with both imaging and surgical equipment and devices, hence providing a realistic experimental testing platform for our prototypes. Different vaginal pressures were simulated by suspending the organ in a custom-made tank that was filled with ultrasound gel of known density, to appropriate heights, to provide the desired pressures through the relation shown in the following equation:

$$\text{Pressure (P)} = \text{density } (\rho) \text{ x acceleration due to gravity (g) x height (h)} \quad \text{Eq. 1}$$

where the density (p) is equal to 1 g/cm3 and acceleration due to gravity (g) is equal to 10 m/s$^2$. Each prototype was inserted into the phantom vagina, opened to the desired position (where applicable) and images of the cervix were captured with a 2MP USB camera (Supereyes Y002) and saved for further analysis to determine the percent visual area (PVA) that each prototype allowed. It should be noted that the 2MP USB camera used for experiments and clinical studies is a low-cost, off-the-shelf camera. The inserters in accordance with disclosed embodiments may be adaptable to the 5 MP colposcope by simply increasing the diameter of the stem diameter of the speculum-free device. Testing was performed under vaginal pressures of 0.1cm $H_2O$ to 15 cm $H_2O$, spanning the range of supine position pressures previously cited. Images were also captured with the standard Graves speculums for comparison. The percentage of unobstructed cervical area, with the os centered, was calculated from each image using a circular grid (30) and Equation 2, which provided a standardized comparison regardless of distance between the camera and the cervix.

$$PVA = (\text{number of square with cervix region} / \text{total number of squares}) \times 100 \qquad \text{Eqn. 2}$$

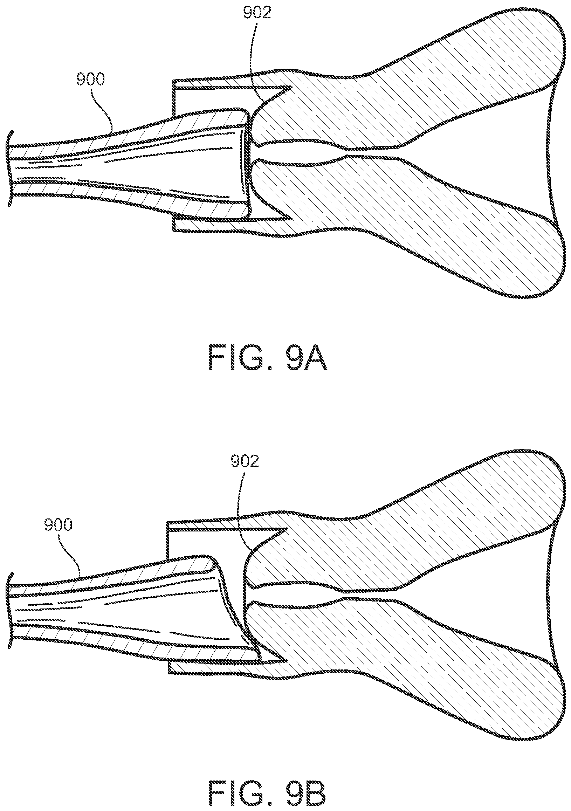

Based on initial favourable testing with the flat tip inserter, an iteration of the inserter with integrated 2MP camera was made for further testing to enable manipulation of the cervix for effective use with women (see Results, Phantom Testing). The probe inserter was further iterated on to enable manipulation of the cervix for effective use in women with severely tilted uteri, namely retroverted (tilted posteriorly), anteverted (tilted anteriorly) and sideverted uteri (tilted to the side) which condition affects about 20% of women. FIGs. 9A and 9B are cross-sectional side views showing using of a flat and curved end Inserters with integrated 2 MP camera 900, respectively, for manipulation of the cervix 902. These figures show how the curved end of the colposcope enables advantageous manipulation of the cervix 902. Since the cervix is shaped like a semi-sphere with the curved portion interfacing with the Inserter, protrusion of the tip of the inserter is required to easily manipulate it. The variation of the design was tested in the phantom to assess ability to manipulate the cervix at different tilts.

To test the two probe Inserters, the uterus in the phantom was tilted under constant pressure (5 cm $H_2O$) 30° to the side for sideverted, 30° downwards for retroverted and 30° upwards for anteverted position. Height and angle measurements were achieved using a 30 cm ruler and a protractor attached to the acrylic walls of the phantom respectively. The prototypes were inserted and used to gently manipulate the cervix into the mid-position, with the os centered as well as possible. Images were then taken for further visual analysis to estimate how much of the cervix, with the os centered, could be visualized. The percentage of unobstructed visual area was calculated using Equation 2. The offset of the os from the center for each image was also determined by measuring the distance from the position of the os to the center of the grid. Each experiment was repeated 5 times and all results were statistically compared between designs and the standard speculum using student t-tests ($\alpha$=0.05).

An IRB-approved clinical study in health volunteers was performed with the flat tip and curved tip inserters to validate the results from the phantom studies. 15 volunteers with informed consent were enrolled to determine feasibility and comfort of the inserter for clinical use, and to validate its ability to view and manipulate the cervix for image capture. The visualization of the cervix and the centeredness of the os for both the flat tip and the curved tip were also qualitatively compared. After informed consent was obtained, volunteers were asked to complete a pre-examination questionnaire to assess their experiences with and attitudes towards the standard speculum and vaginal examinations. Volunteers were then trained for about 5 minutes on how to use the inserter with the camera and capture images of their cervices using a pelvic mannequin. Once trained, each volunteer inserted the device herself and maneuvered it to find her cervix with the aid of the camera (for image capture) and mobile phone (for image display). Images were captured once the cervix was in view. Since these were healthy volunteers, no acetic acid was applied during the procedure. After the examination was complete, the volunteers were asked to complete post-examination questionnaires. The volunteer post-examination questionnaires assessed comfort and compared their experience with the inserter to previous examinations with a speculum.

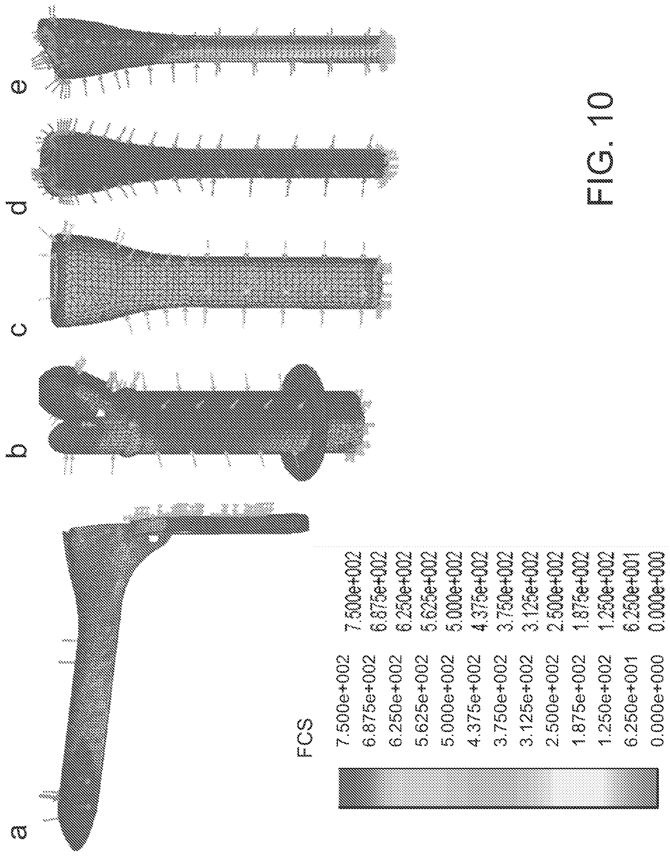

FIG. 10 shows images of a finite element analysis with factor of safety (FOS) plots over the entire device, ranging from relatively low values (lower end of scale on right) to high FOS values (upper end of scale on right). Various inserters as disclosed herein are shown in images (a) - (e) of FIG. 10. The lower the FOS, the more likely the device is to fail under pressure and possibly fracture within the patient's body during use. This metric is especially important for devices that have to expand to open the vaginal walls. All the devices had a minimum FOS above the acceptable value of 4. The standard speculum had a minimum FOS of 8.07. The billed expander was rated 11.67; the silicone expander, 346; the flat tip inserter, 3095.3; and the curved tip inserter, 90.9.

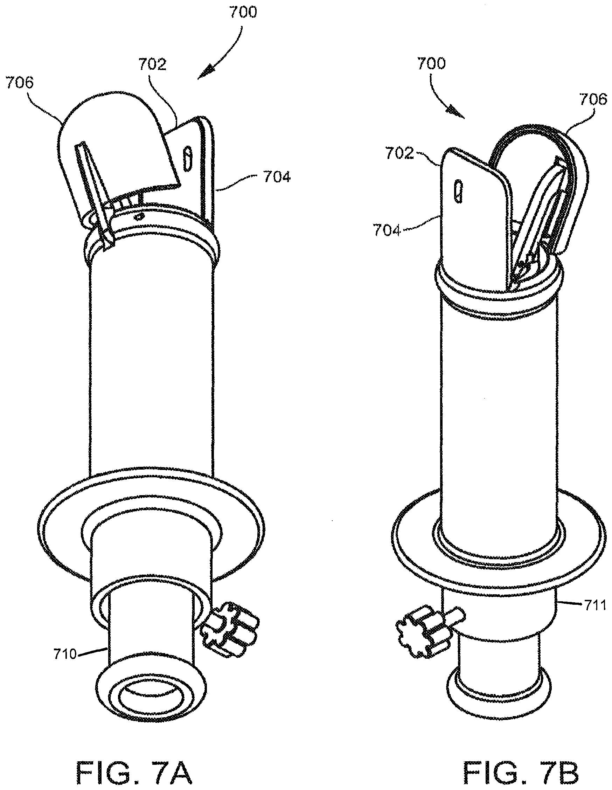

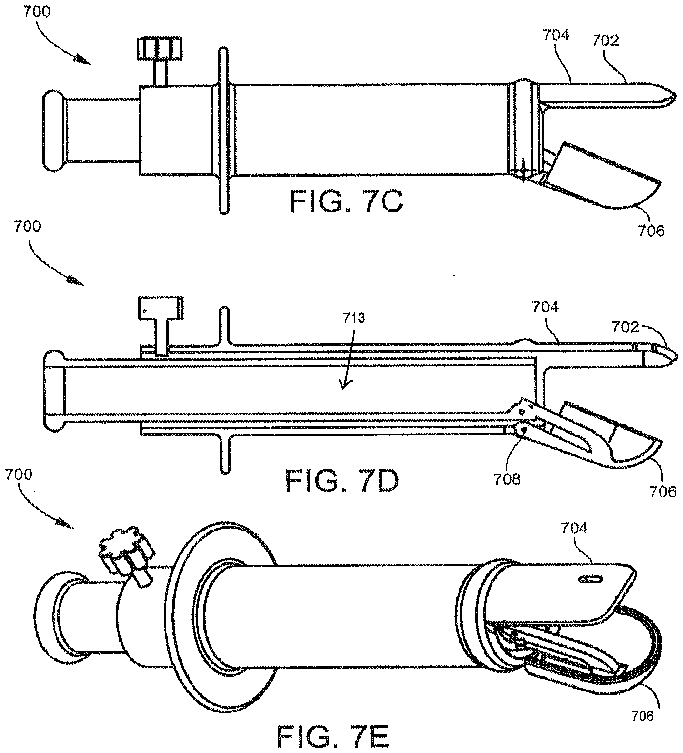

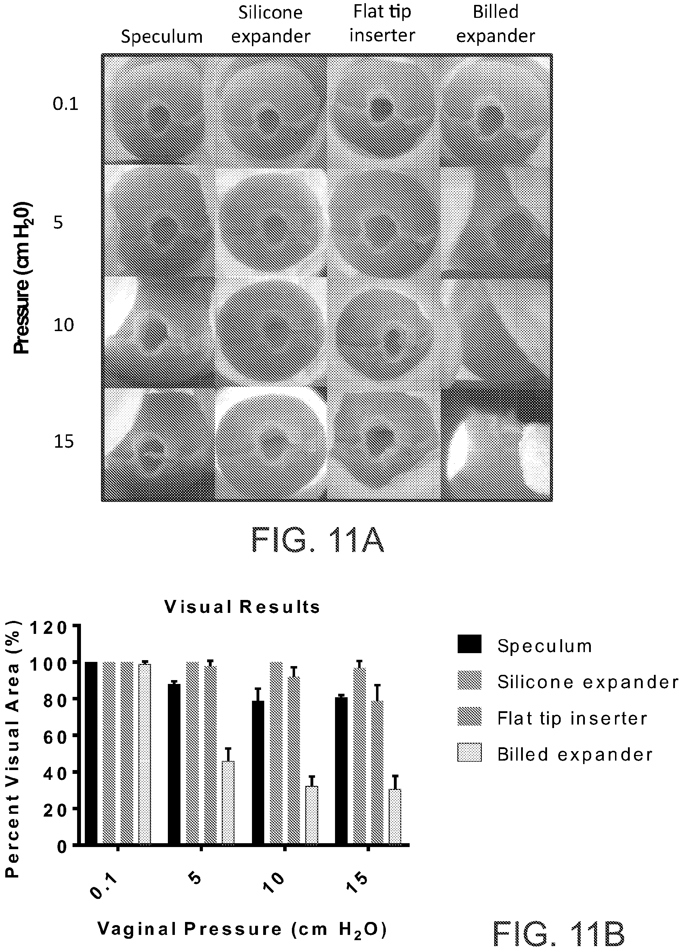

Testing in the phantom was conducted to compare visualization of the cervix afforded by the different prototypes under different pressures (0.1-15 cm $H_2O$) corresponding to and slightly above a range of vaginal pressures of 0.1-12.0 cm $H_2O$ measured in the supine position. Images were also taken with a standard medium-sized Graves speculum to compare visualization with the standard of care. FIG. 11A shows representative images from each prototype as well as the standard Graves speculum of the cervix phantom at an untilted position under different pressures. The images show the central os of the cervix, the cervix and the vaginal walls in some cases. Table 1 shows the mean percent visualization for the speculum, silicone expander, flat tip inserter and the billed expander. FIG. 11B shows a grouped bar plot of mean percent visual area of the cervix for different devices disclosed herein under different pressures. Error bars shown in FIG. 7B are standard deviations.

Table 1

| Pressure value (cm $H_2O$) | 0.1 | 5 | 10 | 15 |
|---|---|---|---|---|
| **Speculum mean PVA (%)** | 100 +/- 0 | 88.1+/-1.5 | 78.8 +/- 6.8 | 80.7 +/- 1.2 |
| **Silicone expander mean PVA (%)** | 100 +/- 0 | 100 +/- 0 | 100 +/- 0 | 96.9 +/- 3.8 |
| **Flat tip inserter mean PVA (%)** | 100 +/- 0 | 97.7 +/- 3.1 | 92.0 +/- 5.3 | 78.86 +/- 8.7 |
| **Billed expander mean PVA (%)** | 98.86 +/-1.5 | 45.78 +/- 7.0 | 31.94 +/-5.7 | 30.38 +/- 7.4 |

Although the devices performed similarly at 0.1 cm $H_2O$, statistically significant differences were found at other pressures. Statistical analysis showed that the mean PVA for the silicone expander was statistically higher (i.e. better) than the standard speculum (p<0.001) for 5, 10 and 15 cm $H_2O$. The PVA for the flat tip inserter was also statistically higher than the standard speculum (p<0.01) for 5 and 10 cm $H_2O$. The billed expander was worse than the speculum (p<0.00001) for 5, 10 and 15 cm $H_2O$. Even though the silicone expander provided the best PVA in these experiments, it was limited by the complexity of insertion and removal.

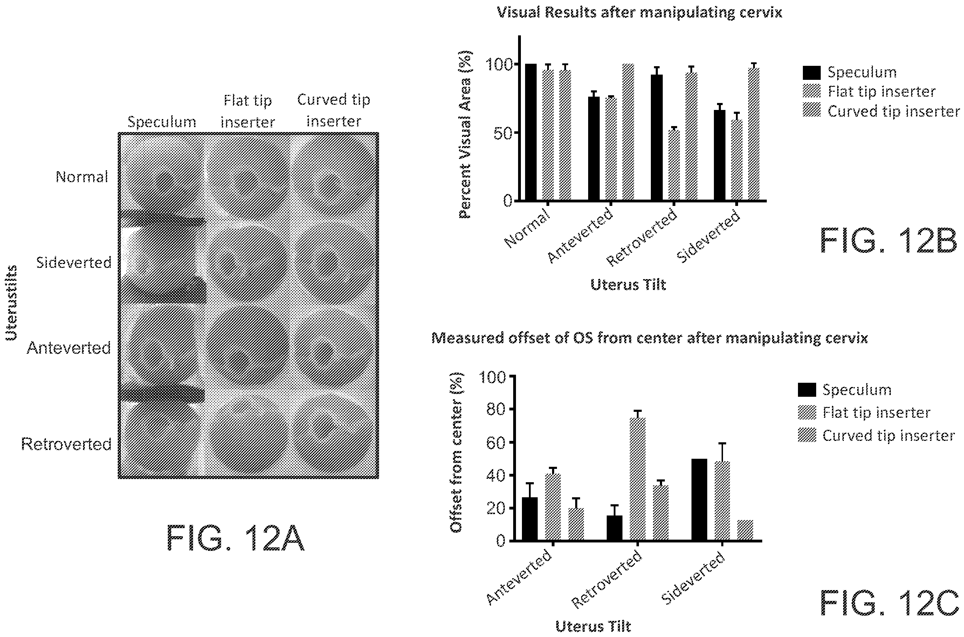

The two variations of the inserter were tested on the vaginal phantom, which had been positioned at different tilts to simulate cervices that are sideverted, anteverted and retroverted. FIG. 12A shows images from testing, after the tilted cervices had been manipulated towards the center position by the Graves speculum and both the flat tip and curved tip inserters. Particularly, FIGs. 12A- 12C show results from comparing the ability of the speculum, flat tip and curved tip probe inserters to manipulate the cervix. FIG. 12A are images of the cervix at after manipulation with the standard speculum, flat tip and curved tip inserters for normal, sideverted, anteverted and retroverted positions. FIG. 12B is a graph showing measured mean offset of the os from the center for each device under different uterus tilts. FIG. 12C is a graph showing mean PVA for each device under different uterus tilts. Error bars are standard deviations.

Table 2 below shows the mean percent visual area (PVA) of the cervix enabled by the speculum, flat tip and curved tip inserters after attempts by each device to center the tilted uterus under constant pressure. Table 3 below shows the mean percent offset of the os from the center after manipulating the cervix. Results showed that the curved tip was best able to manipulate the cervix to a position with the os closest to the center, providing the lowest offsets with p <0.00001 for the sideverted position compared to the speculum and p<0.001 for all three positions compared to the flat tip inserter. The curved tip also provided the highest PVA across the sideverted and anteverted positions compared to the speculum (p<0.001) and across all three positions compared to the flat tip inserter (p<0.00001).

Table 2

| Uterine Position | Normal Position | Anteverted | Retroverted | Sideverted |
|---|---|---|---|---|
| **Speculum mean PVA (%)** | 100 +/- 0 | 75.8 +/-4 | 92.0 +/- 5.3 | 66.5 +/-4 |
| **Flat tip inserter mean PVA (%)** | 95.78 +/- 3.92 | 75+/-1.2 | 51.9 +/- 2.1 | 59.2 +/- 5.4 |
| **Curved tip inserter mean PVA (%)** | 95.4 +/-4.5 | 100 +/-0 | 93.8 +/-4.3 | 96.9 +/- 3.8 |

Table 3

| Uterine Position | Anteverted | Retroverted | Sideverted |
|---|---|---|---|
| **Speculum mean offset (%)** | 26.6+/-8.4 | 15.6+/-6.2 | 50+/-0 |
| **Flat tip inserter mean offset (%)** | 41.0+/-3.6 | 74.7+/-4.4 | 48.5+/-11.0 |
| **Curved tip inserter mean offset (%)** | 20.0+/-6.1 | 33.9+/-3.0 | 12.5+/-0 |

Testing of the flat tip and curved tip inserter variations were performed in fifteen volunteers. Volunteer demographics for the study are outlined in Table 4. For demographics, the number of vaginal deliveries, previous history with the speculum and use of tampons were observed, as these could affect how easy and comfortable women would find the inserter. It was also determined whether women thought the speculum was a barrier to screening and what they considered to be the top three most important factors for cervical cancer screening. Most of the volunteers were under the age of 29 and had not had any vaginal deliveries.

Approximately half of them had had fewer than two speculum examinations, however three- quarters of the volunteers were regular users of tampons/menstrual cups. Twothirds of the women did not consider the speculum to be a barrier to cervical cancer screening, however one-third thought it was a small to medium barrier. Most of the women thought that adequate assessment of risk was most important in cervical cancer screening, while cost (threequarters of the women) and comfort (half of women) were the second and third most important factors, respectively. Only one woman considered physician gender to be an important factor.

Table 4

| Characteristics | Response | Volunteers % (n) |
|---|---|---|
| Age, y | | 86.7 (13) |
| | □ ≤ 29 | 6.7(1) |
| | □ 40-44 | 6.7(1) |
| | □ 45-49 | |
| Number of vaginal deliveries | | 93.3 (14) |
| | □ 0 | 6.7 (1) |
| | □ 3 | |
| Number of cervical examinations with a speculum | | 13.3 (2) |
| | □ 0 | 33.3 (5) |
| | □ 1-2 | 26.7 (4) |
| | □ 3-5 | 6.7 (1) |
| | □ 6-10 | 20 (3) |
| | □ >10 | |
| Regular use of tampons | | 77.8 (7/9) |
| | □ Yes | 22.2 (2/9) |
| | □ No | |
| Barrier of speculum to getting cervical cancer screening | | 60 (9) |
| | □ Not a barrier | 26.7(4) |
| | □ Small barrier | 13.3 (2) |
| | □ Medium barrier | 0(0) |
| | □ Large barrier | |
| Top 3 most important considerations for cervical cancer examination | | 93.3 (14) |
| | □ Adequate assessment of risk | 46.7 (7) |
| | □ Travel distance to provider | 73.3 (11) |
| | □ Cost | 53.3 (8) |
| | □ Comfort during screening | 26.7 (4) |
| | □ Procedure time | 6.7 (1) |
| | □ Physician gender | |

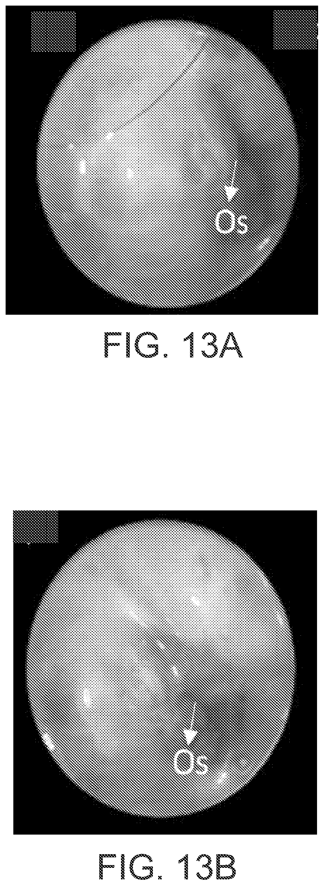

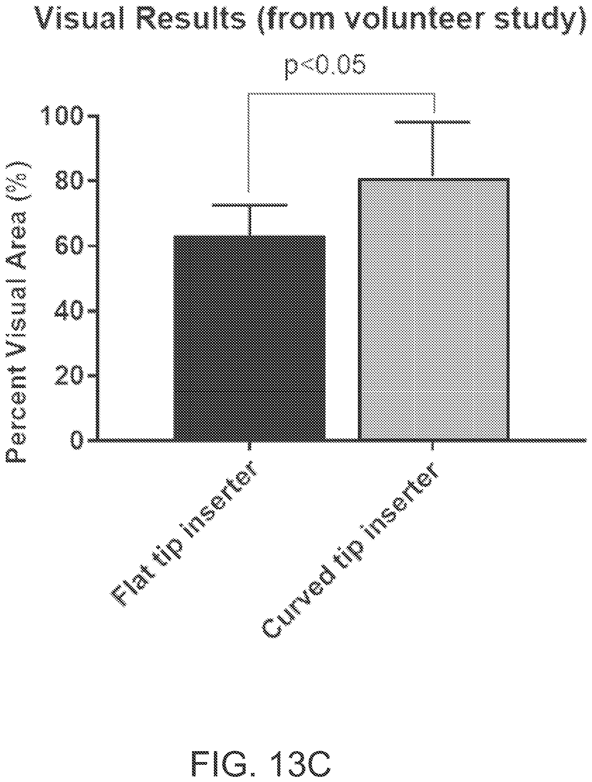

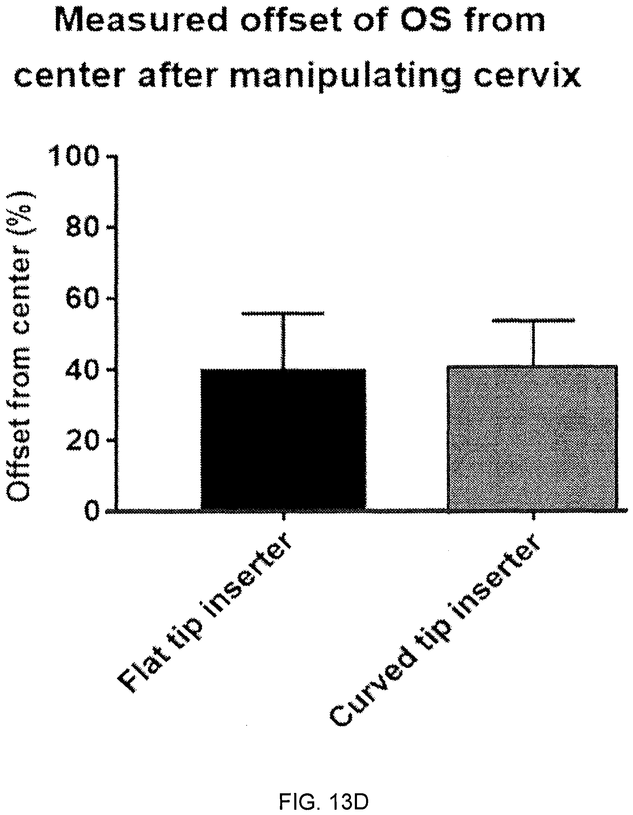

Images were captured from twelve out of the fifteen volunteers. Six of the acquired images were obtained from the flat tip inserter while six of the images were acquired from the curved tip inserter. FIGs. 13A and 13B show representative images from a single volunteer with a sideverted cervix using the flat tip inserter (FIG. 13A) and the curved tip inserter (FIG. 13B). The flat tip inserter was only able to sufficiently manipulate the cervix to a centered position and provide an adequate view (constitutes the entire os and a proportion of the surrounding cervix on all sides of the os) of the cervix for two out of six of the women assigned to it. In contrast, the curved tip inserter was able to center the cervix for imaging and provide an adequate view of the cervix for five out of six women, showing the os and an adequate section of the cervix. The mean percent visual area for the six patients who used the flat tip inserter was 63.4 +/- 9.1% and the mean percent offset was 40.25 +/- 15.6%. For the curved tip inserter, the mean percent visual area 81.5+/- 16.7% and the mean percent offset was 38.8+/-15% (FIGs. 13C and 13D).

The body 506 can define an internal space or channel through which a camera (not shown) with LED illumination (e.g., a 2MP mini USB camera) to enable cervix image capture. The light and camera feature allows for an image of the cervix to be lit and captured. Further, the internal space of the body 506 or channel may also enable acetic acid/Lugol's iodine application and insertion of swabs for Pap smear sample collection. The camera can be connected to the electronic device 502 for image capture. The colposcope 100 can provide for patientcentered colposcopy and can also be used to center and identify the cervix for physician-based or self-Pap/HPV testing.

FIGs. 6A and 6B illustrate a perspective view and a cross-sectional side view, respectively of a body of the colposcope 500 shown in FIG. 5. Referring particularly, to FIG. 6B, the body may form an interior channel 522 or other interior space. An image capture device, such as a waterproof, 2 mega pixel, Supereyes Y002, may be suitably positioned and attached inside the channel 522. In an example, the camera may have four white LEDs for illumination and a lens covered by a plane hydrophobic window. The associated colposcope may be configured to have adjustable brightness and a manual focus. Electronics for image capture and lighting may be housed in a metallic body of the image capture device (e.g., camera). interfaces via a USB cable to a phone, tablet, or computer, all of which provide power to the camera and enable image capture. Thus, the camera may only require a charged phone, tablet, or computer to operate, but does not require AC power or a separate battery source. The cross-sectional view of the inserter shows an increasing thickness from the bottom to the tip. This enables preservation of the slim body while enabling a blunt, comfortable tip for introduction into the patient. The slanted curve of the tip also enables a gradual and hence more comfortable introduction as compared to a flat tip.

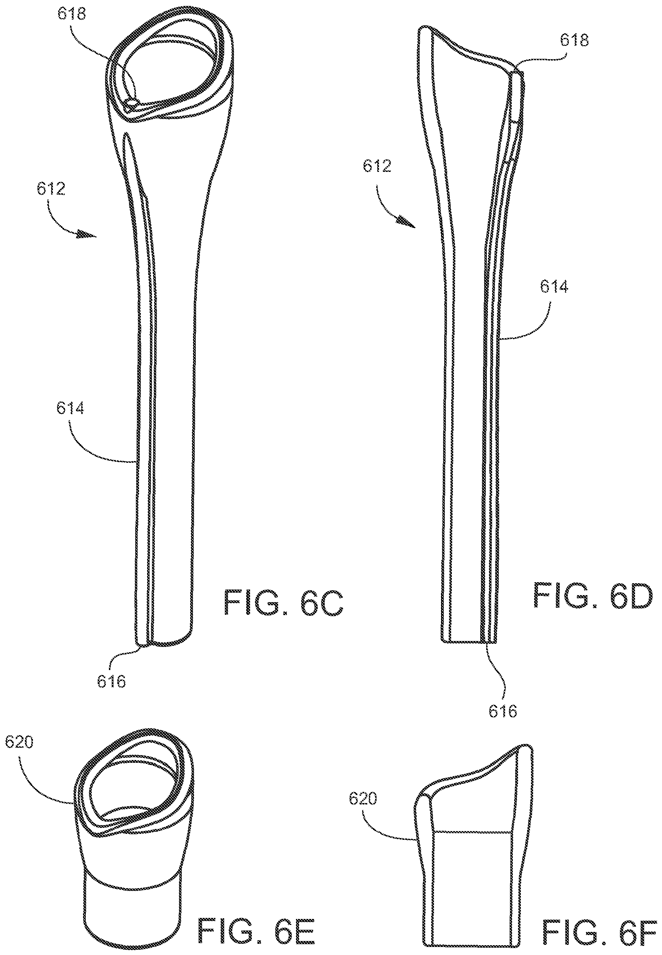

In accordance with other embodiments, FIGs. 6C and 6D are a perspective view and a cross-sectional side view, respectively, of another example inserter 612. Referring to FIGs. 6C and 6D, the inserter 612 is similar to the inserter shown in FIGs. 6A and 6B. Inserter 612 differs in that it also includes a channel 614 that extends a length of the inserter 612. The channel 614 includes an opening 616 at a proximate end of the inserter 612. The channel also includes another opening 618 at a distal end of the inserter 612. The openings 614 and 616 are in fluid communication such that fluid can be moved between the openings 614 and 616. In an example, opening 616 may be suitably connected to a source of acetic acid. The acetic acid may be suitable moved from the source and through the channel 614 to the opening 616 for spray on an organ tissue bed of interest for imaging.

FIGs. 6E and 6F illustrate a perspective view and a cross-sectional side view, respectively, of another example inserter 620 in accordance with embodiments of the present disclosure. Referring to FIGs. 6E and 6F, the inserter 620 is similar to the inserter 500 shown in FIG. 5. The inserter 620 shown in FIGs. 6E and 6F differs in that it consists of the tip of the inserter 600 modified to fit on the tip of the colposcope 100 for speculum-free cervix imaging.

Other than the curved funnel-like tip embodiment shown in FIG. 5, various alternative inserters are disclosed herein. FIGs. 7A - 7E illustrate different views of another inserter referred to herein as the mechanical billed expander 700 for use with a colposcope in a closed configuration and open configuration, respectively, in accordance with embodiments of the present disclosure. FIGs. 7A- 7E depict a bottom perspective view, a top perspective view, a side view, a cross-sectional side view, and another top perspective view. Referring to FIGs. 7A - 7E, the expander 700 includes a distal end 702 having a pair of bills 704 and 706 that can be closed together upon insertion and opened once inserted to expand. The bills 704 and 706 can open by rotation of the bill 706 about a pivot 708. The bill 706 may be rotated by pushing a plunger connected to an extension 710 of the bill that causes rotation of the bill 706 about the pivot point 708. A screw knob can be twisted to lock the bills 704 and 706 in position once opened to the desired diameter. The expander 700 also includes a handle 711. The expander 700 may also be operatively connected to a device similar to the device 502 shown in FIG. 5 for controlling the operation of the colposcope 500, processing captured images, and interfacing with a user. An image capture device, such as a waterproof, 2 mega pixel, Supereyes Y002 camera, may be positioned and fitted in an interior channel 713 of the expander. In an example, the image capture device may have four white LEDs for illumination and a lens covered by a plane hydrophobic window. The LEDs may have adjustable brightness and a manual focus. Electronics for image capture and lighting may be housed in a metallic body of the camera. The electronics may interface via a USB cable to a phone, tablet, or computer, all of which provide power to the camera and enable image capture. Thus, the camera only requires a charged phone, tablet, or computer to operate, but does not require AC power or a separate battery source.

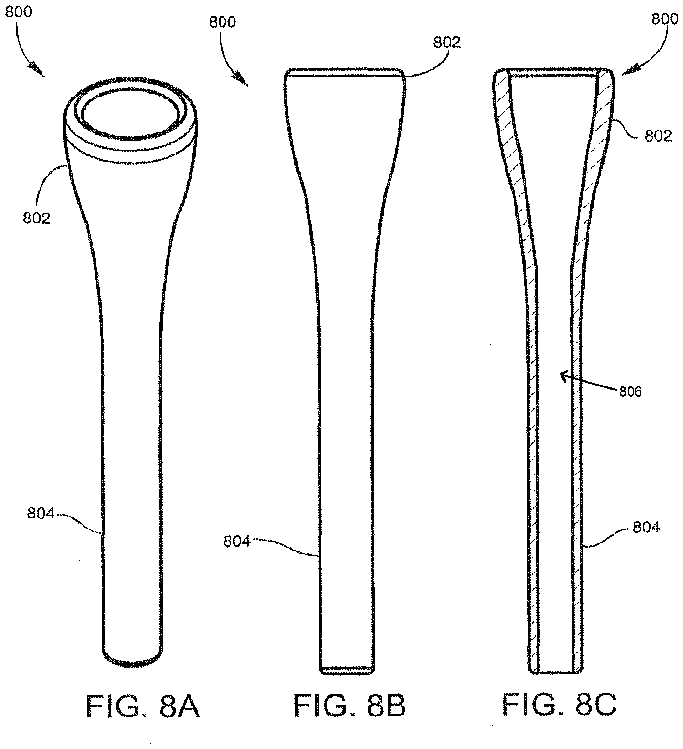

In another example, FIGs. 8A - 8C illustrate different views of a flat-tip inserter 800 for use with a colposcope in accordance with embodiments of the present disclosure. Referring to FIG. 8, the inserter 800 includes a distal end 802 having a flat tip. The inserter 800 also includes a handle 804. The inserter 800 may be similar to the body shown in FIGs. 6A and 6B except that the tip is flat rather than curved. An image capture device, such as a waterproof, 2 mega pixel, Supereyes Y002 camera, may be positioned and fitted in an interior channel 806 of the inserter. In an example, the image capture device may have four white LEDs for illumination and a lens covered by a plane hydrophobic window. The LEDs may have adjustable brightness and a manual focus. Electronics for image capture and lighting may be housed in a metallic body of the camera. The electronics may interface via a USB cable to a phone, tablet, or computer, all of which provide power to the camera and enable image capture. Thus, the camera only requires a charged phone, tablet, or computer to operate, but does not require AC power or a separate battery source.

It is noted that in the embodiments of the inserter disclosed herein, the inserter may define an interior space that can serve as a working channel for camera placement, acetic acid application, and other features.

The electronic device 502 may be configured to control the operation of the colposcope 500, to process captured images, and to interface with a user, such as medical personnel. In this example, the electronic device 502 is a smartphone, although it should be understood that the electronic device 502 may alternatively be any other type of computing device. It is noted that the term "electronic device" should be broadly construed. It can include any type of device capable of presenting electronic text to a user. For example, the electronic device may be a mobile device such as, for example, but not limited to, a smart phone, a cell phone, a pager, a personal digital assistant (PDA, e.g., with GPRS NIC), a mobile computer with a smart phone client, or the like. An electronic device can also include any type of conventional computer, for example, a desktop computer or a laptop computer. A typical mobile device is a wireless data access-enabled device (e.g., an iPHONE® smart phone, a BLACKBERRY® smart phone, a NEXUS ONE™ smart phone, an iPAD® device, or the like) that is capable of sending and receiving data in a wireless manner using protocols like the Internet Protocol, or IP, and the wireless application protocol, or WAP. This allows users to access information via wireless devices, such as smart phones, mobile phones, pagers, two-way radios, communicators, and the like. Wireless data access is supported by many wireless networks, including, but not limited to, CDPD, CDMA, GSM, PDC, PHS, TDMA, FLEX, ReFLEX, iDEN, TETRA, DECT, DataTAC, Mobitex, EDGE and other 2G, 3G, 4G and LTE technologies, and it operates with many handheld device operating systems, such as PalmOS, EPOC, Windows CE, FLEXOS, OS/9, JavaOS, iOS and Android. Typically, these devices use graphical displays and can access the Internet (or other communications network) on so-called mini- or micro-browsers, which are web browsers with small file sizes that can accommodate the reduced memory constraints of wireless networks. In a representative embodiment, the mobile device is a cellular telephone or smart phone that operates over GPRS (General Packet Radio Services), which is a data technology for GSM networks. In addition to a conventional voice communication, a given mobile device can communicate with another such device via many different types of message transfer techniques, including SMS (short message service), enhanced SMS (EMS), multi-media message (MMS), email WAP, paging, or other known or later-developed wireless data formats. Example functions described herein may be implemented on any suitable electronic device, such as a computer or smartphone.

The electronic device 502 may include a touchscreen display 520 and/or other user interface for interacting with a user and for present information and images. As referred to herein, a "user interface" (UI) is generally a system by which users interact with an electronic device. An interface can include an input for allowing users to manipulate an electronic device, and can include an output for allowing the system to present information (e.g., e-book content) and/or data, indicate the effects of the user's manipulation, etc. An example of an interface on an electronic device includes a graphical user interface (GUI) that allows users to interact with programs in more ways than typing. A GUI typically can offer display objects, and visual indicators, as opposed to text-based interfaces, typed command labels or text navigation to represent information and actions available to a user. For example, an interface can be a display window or display object, which is selectable by a user of a mobile device for interaction. The display object can be displayed on a display screen of an electronic device and can be selected by and interacted with by a user using the interface. In an example, the display of the electronic device can be a touch screen, which can display the display icon. The user can depress the area of the display screen at which the display icon is displayed for selecting the display icon. In another example, the user can use any other suitable interface of a mobile device, such as a keypad, to select the display icon or display object. For example, the user can use a track ball or arrow keys for moving a cursor to highlight and select the display object.

Operating environments in which embodiments of the present disclosure may be implemented are also well-known. The electronic device 502 may be communicatively connected to a remote server for communication of data and captured images for processing in accordance with embodiments of the present disclosure. Further, the electronic device 502 may suitably power the light emitters 516 and the image capture device 518 via the cable 504. In a representative embodiment, an electronic device, such as an e-book reader, is connectable (for example, via WAP) to a transmission functionality that varies depending on implementation. Thus, for example, where the operating environment is a wide area wireless network (e.g., a 2.5G network, a 3G network, or a 4G network), the transmission functionality comprises one or more components such as a mobile switching center (MSC) (an enhanced ISDN switch that is responsible for call handling of mobile subscribers), a visitor location register (VLR) (an intelligent database that stores on a temporary basis data required to handle calls set up or received by mobile devices registered with the VLR), a home location register (HLR) (an intelligent database responsible for management of each subscriber's records), one or more base stations (which provide radio coverage with a cell), a base station controller (BSC) (a switch that acts as a local concentrator of traffic and provides local switching to effect handover between base stations), and a packet control unit (PCU) (a device that separates data traffic coming from a mobile device). The HLR also controls certain services associated with incoming calls. Of course, embodiments in accordance with the present disclosure may be implemented in other and next-generation mobile networks and devices as well. The mobile device is the physical equipment used by the end user, typically a subscriber to the wireless network. Typically, a mobile device is a 2.5G-compliant device, 3G-compliant device, or 4G-compliant device that includes a subscriber identity module (SIM), which is a smart card that carries subscriber-specific information, mobile equipment (e.g., radio and associated signal processing devices), a user interface (or a man-machine interface (MMI)), and one or more interfaces to external devices (e.g., computers, PDAs, and the like). The electronic device may also include a memory or data store.

Any patents or publications mentioned in this specification are indicative of the levels of those skilled in the art to which the present subject matter pertains.

One skilled in the art will readily appreciate that the present subject matter is well adapted to carry out the objects and obtain the ends and advantages mentioned, as well as those inherent therein. The present examples along with the methods described herein are presently representative of various embodiments, are exemplary, and are not intended as limitations on the scope of the present subject matter. Changes therein and other uses will occur to those skilled in the art which are encompassed within the present subject matter as defined by the scope of the claims.

**Claims**

1.  A colposcope (100) comprising:

    an elongate body (102, 128) defining an interior space and having a distal end (106), a proximate end (104), and an axis extending between the distal end (106) and the proximate end (104);
    an image capture device (132) positioned within the interior space of the elongate body;
    at least one light emitter attached to the distal end (106) of the elongate body and positioned to generate and direct light towards the area outside of the elongate body, wherein the at least one light emitter comprises a plurality of light emitting diodes (LEDs);
    an anti-reflection coated, hydrophobic window positioned at the distal end and positioned within a field of view of the image capture device (132);
    **characterized in that** the colposcope further comprises:

    a control panel (110, 112) on the body and associated electronics configured to select from among a plurality of modes of illumination for an object in a field of view of the image capture device,
    wherein the control panel (110, 112) and associated electronics are configured to select among the modes of illumination to control the plurality of LEDs.

2.  The colposcope of claim 1, wherein the plurality of LEDs comprises a plurality of different types of light emitting diodes (LEDs), and wherein the modes of illumination correspond to activation and deactivation of the different types of LEDs.

3.  The colposcope of claim 1, wherein the control panel (110, 112) and associated electronics are configured to select from among a plurality of different magnifications or colors for viewing an object in a field of view of the image capture device (132).

4.  The colposcope of claim 1, wherein the window is configured to reduce glare and provide impact and abrasion protection, and form the outermost liquid and particle barrier to the sensitive camera lens.

5.  The colposcope of claim 1, wherein the body comprises first and second housing parts (114, 116) that are ultrasonically welded to provide a liquid and particle tight seal.

6.  The colposcope of claim 1, further comprising a light guide and diffuser 137 )located in the distal end of the colposcope.

7.  The colposcope of claim 6, wherein the plurality of LEDs are a concentric LED PCB ring and the light guide and diffuser (133) structurally supports the concentric LED PCB ring.

8.  The colposcope of claim 1, further comprising a modular reflector to provide uniform illumination by redirecting light at outer fringes back towards target tissue surface through the use of angled sidewalls of the distal end.

9.  The colposcope of claim 1, wherein the LEDs are configured for one of white light imaging for visual inspection with acetic acid (VIA) and Lugol's iodine (VILLI), green light imaging for enhanced contrast for vasculature, and blue light imaging for excitation illumination of potential target fluorophores.

**Patentansprüche**

1.  Ein Kolposkop (100), das Folgendes beinhaltet:

einen länglichen Hauptteil (102, 128), der einen Innenraum definiert und ein distales Ende (106), ein proximales Ende (104) und eine Achse aufweist, die sich zwischen dem distalen Ende (106) und dem proximalen Ende (104) erstreckt;

eine innerhalb des Innenraums des länglichen Hauptteils positionierte Bilderfassungsvorrichtung (132);

mindestens einen Lichtsender, der an dem distalen Ende (106) des länglichen Hauptteils angebracht ist und so positioniert ist, dass er Licht erzeugt und auf den Bereich außerhalb des länglichen Hauptteils lenkt, wobei der mindestens eine Lichtsender eine Vielzahl von Leuchtdioden (LEDs) beinhaltet;

ein antireflexbeschichtetes, hydrophobes Fenster, das an dem distalen Ende positioniert ist und innerhalb eines Sichtfelds der Bilderfassungsvorrichtung (132) positioniert ist;

**dadurch gekennzeichnet, dass** das Kolposkop ferner Folgendes beinhaltet:

ein Bedienfeld (110, 112) an dem Hauptteil und dazugehörige Elektronik, die dazu ausgelegt ist, aus einer Vielzahl von Beleuchtungsmodi für einen Gegenstand in einem Sichtfeld der Bilderfassungsvorrichtung auszuwählen,

wobei das Bedienfeld (110, 112) und die dazugehörige Elektronik dazu ausgelegt sind, aus den Beleuchtungsmodi zu wählen, um die Vielzahl von LEDs zu steuern.

2. Kolposkop gemäß Anspruch 1, wobei die Vielzahl von LEDs eine Vielzahl verschiedener Typen von Leuchtdioden (LEDs) beinhaltet und wobei die Beleuchtungsmodi der Aktivierung und Deaktivierung der verschiedenen Typen von LEDs entsprechen.

3. Kolposkop gemäß Anspruch 1, wobei das Bedienfeld (110, 112) und die dazugehörige Elektronik dazu ausgelegt sind, aus einer Vielzahl verschiedener Vergrößerungen oder Farben zur Ansicht eines Gegenstands in einem Sichtfeld der Bilderfassungsvorrichtung (132) auszuwählen.

4. Kolposkop gemäß Anspruch 1, wobei das Fenster dazu ausgelegt ist, die Blendung zu verringern und Aufprall- und Abriebschutz bereitzustellen und die äußerste Flüssigkeits- und Partikelbarriere vor der empfindlichen Kameralinse zu bilden.

5. Kolposkop gemäß Anspruch 1, wobei der Hauptteil einen ersten und zweiten Gehäuseteil (114, 116) beinhaltet, die ultraschallverschweißt sind, um eine flüssigkeits- und partikeldichte Abdichtung bereitzustellen.

6. Kolposkop gemäß Anspruch 1, das ferner einen am distalen Ende des Kolposkops befindlichen Lichtleiter und -diffusor (137) beinhaltet.

7. Kolposkop gemäß Anspruch 6, wobei die Vielzahl von LEDs ein konzentrischer LED-PCB-Ring sind und der Lichtleiter und -diffusor (133) den konzentrischen LED-PCB-Ring strukturell stützt.

8. Kolposkop gemäß Anspruch 1, das ferner einen modularen Reflektor beinhaltet, um eine gleichmäßige Beleuchtung bereitzustellen, indem das Licht an den äußeren Rändern anhand abgewinkelter Seitenwände des distalen Endes wieder zu der Oberfläche des Zielgewebes zurückgeleitet wird.

9. Kolposkop gemäß Anspruch 1, wobei die LEDs für eine der Folgenden ausgelegt sind: Weißlichtbildgebung zur Sichtprüfung mit Essigsäure (VIA) und Lugol-Iod (VILLI), Grünlichtbildgebung für einen erhöhten Kontrast für das Gefäßsystem und Blaulichtbildgebung für die Anregungsbeleuchtung potenzieller Zielfluorophore.

**Revendications**

1. Colposcope (100) comprenant :

un corps allongé (102, 128) définissant un espace intérieur et présentant une extrémité distale (106), une extrémité proximale (104), et un axe s'étendant entre l'extrémité distale (106) et l'extrémité proximale (104) ;

un dispositif de capture d'images (132) positionné au sein de l'espace intérieur du corps allongé ;

au moins un émetteur de lumière fixé à l'extrémité distale (106) du corps allongé et positionné pour générer et diriger une lumière vers la zone en dehors du corps allongé, dans lequel l'au moins un émetteur de lumière comprend une pluralité de diodes électroluminescentes (LED) ;

une fenêtre hydrophobe à revêtement antireflet positionnée au niveau de l'extrémité distale et positionnée dans un champ de vision du dispositif de capture d'images (132) ;
**caractérisé en ce que** le colposcope comprend en outre :

un panneau de commande (110, 112) sur le corps et de l'équipement électronique associé configurés pour sélectionner parmi une pluralité de modes d'éclairage pour un objet dans un champ de vision du dispositif de capture d'images,
dans lequel le panneau de commande (110, 112) et l'équipement électronique associé sont configurés pour sélectionner parmi des modes d'éclairage pour commander la pluralité de LED.

2. Colposcope selon la revendication 1, dans lequel la pluralité de LED comprend une pluralité de différents types de diodes électroluminescentes (LED), et dans lequel les modes d'éclairage correspondent à une activation et une désactivation des différents types de LED.

3. Colposcope selon la revendication 1, dans lequel le panneau de commande (110, 112) et l'équipement électronique associé sont configurés pour sélectionner parmi une pluralité de différents grossissements ou couleurs pour visualiser un objet dans un champ de vision du dispositif de capture d'images (132).

4. Colposcope selon la revendication 1, dans lequel la fenêtre est configurée pour réduire un reflet et fournir une protection contre l'abrasion et les chocs, et pour former une barrière aux liquides et particules la plus externe pour la lentille de caméra sensible.

5. Colposcope selon la revendication 1, dans lequel le corps comprend une première et une seconde parties de boîtier (114, 116) qui sont soudées par ultrasons pour fournir un joint étanche aux liquides et aux particules.

6. Colposcope selon la revendication 1, comprenant en outre un guide et un diffuseur de lumière (137) situé dans l'extrémité distale du colposcope.

7. Colposcope selon la revendication 6, dans lequel la pluralité de LED sont un anneau PCB à LED concentrique et le guide et diffuseur de lumière (133) supporte de manière structurelle l'anneau PCB à LED concentrique.

8. Colposcope selon la revendication 1, comprenant en outre un réflecteur modulaire pour fournir un éclairage uniforme en redirigeant une lumière au niveau de bordures externes vers une surface de tissu cible par l'utilisation de parois latérales inclinées de l'extrémité distale.

9. Colposcope selon la revendication 1, dans lequel les LED sont configurées pour une d'une imagerie à la lumière blanche pour une inspection visuelle avec de l'acide acétique (VIA) et de la solution iodée de Lugol (VILLI), d'une imagerie à la lumière verte pour un contraste amélioré de la vascularisation, et d'une imagerie à la lumière bleue pour un éclairage d'excitation de fluorophores cibles potentiels.

FIG. 1A

FIG. 1B

FIG. 1C

FIG. 1D

EP 3 435 837 B1

FIG. 1E

FIG. 1F

FIG. 1G

FIG. 1H

FIG. 1I

FIG. 1J

FIG. 1K

130

140

136

138

FIG. 1L

130

140

136

138

FIG. 1M

**75° Reflector**

$1.20*10^{-3}$ mw/cm$^2$

$6.00*10^{-4}$

FIG. 2A

FIG. 2B

FIG. 2C

FIG. 2D

FIG. 2E

EP 3 435 837 B1

FIG. 3

FIG. 4

FIG. 5

FIG. 6A

FIG. 6B

FIG. 6C

FIG. 6D

FIG. 6E

FIG. 6F

FIG. 7A

FIG. 7B

700

704 702

FIG. 7C

706

700

713 704

702

FIG. 7D

708 706

700

704

FIG. 7E

706

FIG. 8A　　　FIG. 8B　　　FIG. 8C

FIG. 9A

FIG. 9B

FIG. 10

FIG. 11A

Visual Results

Vaginal Pressure (cm $H_2O$)

FIG. 11B

FIG. 12A

FIG. 12B

FIG. 12C

FIG. 13A

FIG. 13B

FIG. 13C

# Measured offset of OS from center after manipulating cervix

FIG. 13D

**EP 3 435 837 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009029254 A1
- WO 2015077684 A1
- US 2005049509 A1